# EUROPEAN PATENT SPECIFICATION

(11) **EP 1 421 093 B1**
(45) Date of publication and mention of the grant of the patent: **29.06.2005**
(21) Application number: 02768713.6
(22) Date of filing: 26.08.2002
(51) Int. Cl.: C07H 15/00

(54) **CRYSTALINE CLINDAMYCIN FREE BASE**
KRISTALLINE FREIE BASE VON CLINDAMYCIN
CLINDAMYCINE CRISTALLINE SOUS FORME DE BASE LIBRE

(30) Priority: 28.08.2001 US 315375 P; 01.05.2002 US 377892 P
(43) Date of publication of application: 26.05.2004
(73) Proprietor: Pharmacia Corporation, St. Louis, MI 63167 (US)
(72) Inventor: CHAO, Robert, S., Santa Clara, CA 95054 (US); HAWLEY, Michael, Kalamazoo, MI 49009 (US); REEDER, Lisa, M., Kalamazoo, MI 49009 (US); JONES, Donald, P., Portage, MI 49024 (US)
(74) Representative: Motion, Keith Robert
(86) International application number: PCT/US2002/027159
(87) International publication number: WO 2003/020736

(56) References cited:
- US-A- 3 475 407
- US-A- 3 496 163
- US-A- 4 895 934
- BIRKENMEYER ET AL.: "Lincomycin. XI. Synthesis ans strucutre of clindamycin a potent antibacterial agent." J. OF MEDICINAL CHEMISTRY, vol. 13, no. 4, 1970, pages 616-619, XP002218285 cited in the application

## Description

This invention relates to the free base of the antibiotic drug clindamycin and, more particularly, to crystalline clindamycin free base and different crystalline forms thereof. The invention also relates to methods of producing such material, and to methods of using such material in pharmaceutical compositions for the treatment and prevention of bacterial infections.

Clindamycin is an antibiotic used to treat a wide variety of bacterial infections. The compound is also known as 7(S)-chloro-7-deoxylincomycin, methyl 7-chloro-6,7,8-trideoxy-6-(1-methyl-trans-4-propyl-L-2-pyrrolidinecarboxamido)-1-thio-L-threo-α-D-galacto-octo-pyranoside or methyl 7-chloro-6,7,8-trideoxy-6-[[(1-methyl-4-propyl-2-pyrrolidinyl)carbonyl]amino]-1-thio-L-threo-α-D-galacto-octo-pyranoside.

The structure of clindamycin is as shown below:

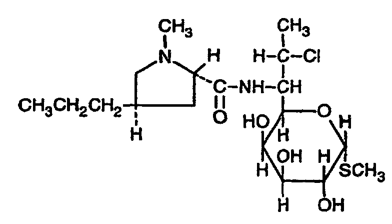

Various methods of synthesis, properties, and uses of clindamycin are set forth in U.S. Patent 3,969,516, Stoughton, issued 1976; U.S. Patent 3,475,407, Bierkenmeyer, issued in 1969; U.S. Patent 3,509,127 and 3,544,551, Kagan and Magerlein, issued in 1970; U.S. Patent 3,513,155, Bierkenmeyer et al , issued in 1970; Morozowich and Sinkula, U.S. Patent 3,508,904 issued in 1971 and 3,655,885 issued in 1972; U.S. Patent 3,714,141, issued in 1973; U.S. Patent 4,568,741 inventor issued in 1986; U.S. Patent 4,710,565 inventor issued in 1984; and WO 01/10407 published in 2001.

Clindamycin is currently used as a salt, such as clindamycin hydrochloride, or as an ester, clindamycin phosphate. Salts and esters of clindamycin can be isolated in crystalline form and these are useful for many applications. Clindamycin hydrochloride is highly water soluble and absorption following oral administration is rapid and nearly complete. The rapid and nearly complete absorption coupled with a half life of about 2.4 hours results in the drug being optimally administered at 6- to 8-hour intervals. Although this can provide rapid and effective treatment of bacterial infections, in some instances formulations which provide a different pharmacokinetic profile such as, for example, one which would allow a longer dosage interval might be desirable.

Clindamycin free base, an amorphous form, has been produced as an end product or as an intermediate product in the synthesis of clindamycin hydrochloride. For example, some references disclose the synthesis of clindamycin free base from lincomycin followed by conversion of the free base of clindamycin to the hydrochloride salt and crystallization of the salt (see for example, U.S. Patent No. 3,475,407; U.S. Patent No. 3,496,163). The free base formed in these processes is the amorphous free base which is characterized as a yellow amorphous solid (see U.S. Patent No. 3,496,163, Example 1) or as a clear, colorless glass (see U.S. Patent No. 3.475,407, Example 1). In the procedures used in these patents, it is indicated that the free base and hydrochloride salt can be subjected to purification procedures such as solvent extractions and washings and chromatography as well as interconversion between the salt and free base, notwithstanding such manipulations of the free base, the free base material is always described as being an amorphous solid. No crystalline free base is described in these patents.

Amorphous Clindamycin free base is relatively insoluble in water compared to clindamycin hydrochloride. Although, the amorphous free base of clindamycin has an antibacterial spectrum of the same scope and magnitude as the hydrochloride salt of clindamycin (see for example, U.S. Patent No. 3,496,163, Example 2), no pharmaceutical preparations of clindamycin free base have, heretofore, been reported in the literature as having been used to treat patients. The absence of reports on the preparation and use of pharmaceutical compositions of amorphous clindamycin free base may be, at least in part to the fact that amorphous materials, in general, are known to have several disadvantages in pharmaceutical development. Amorphous solids, due to their lack of an organized, lattice-like structures, are less energetically stable. The energy required for a drug molecule to escape from a crystal, for example, is greater than is required for the same drug molecule to escape from a non-crystalline, amorphous compound. Due to this fact amorphous solids usually have a faster dissolution rate, a higher apparent solubility, and a lower chemical and physical stability than crystalline materials of the same compound. Another common disadvantage of amorphous solids is a more pronounced hygroscopicity. Yet another disadvantage of amorphous solids is that they are often more difficult to process as dry powders than corresponding powders consisting of crystalline particles, as they are more prone to form aggregates and do not flow as readily.

Birkenmeyer et al. *(J. Med. Chem 13*:616-619, 1970) briefly mentions crystallization of clindamycin free base, but fails to provide any details which would allow one to perform such a crystallization. This reference describes three procedures that can be used in the synthesis of clindamycin, all of which yielded a free base, which can then be converted into the hydrochloride salt. The free base so formed was characterized as an "amorphous solid". The reference further describes the "crystallization" of a portion of the amorphous solid from ethanol-water to give clindamycin free base which was then characterized as to optical rotation and analytical composition. However, no test results were reported therein to indicate what portions, if any, of the crystalized amorphous solid was crystalline free base. Furthermore, the methods used in the crystallization procedure were not described in sufficient detail such that they could be repeated, so the end product could be analyzed..

Thus, although the free base clindamycin could potentially form the basis of new formulations as a result of its being water-insoluble in contrast to the water solubility of the hydrochloride salt of clindamycin, pharmaceutical compositions of clindamycin formulated with the free base have not been made. This is because crystalline clindamycin free base, which the inventors herein believe to be the preferred pharmaceutical form, has not been available heretofore.

### BRIEF DESCRIPTION OF THE INVENTION

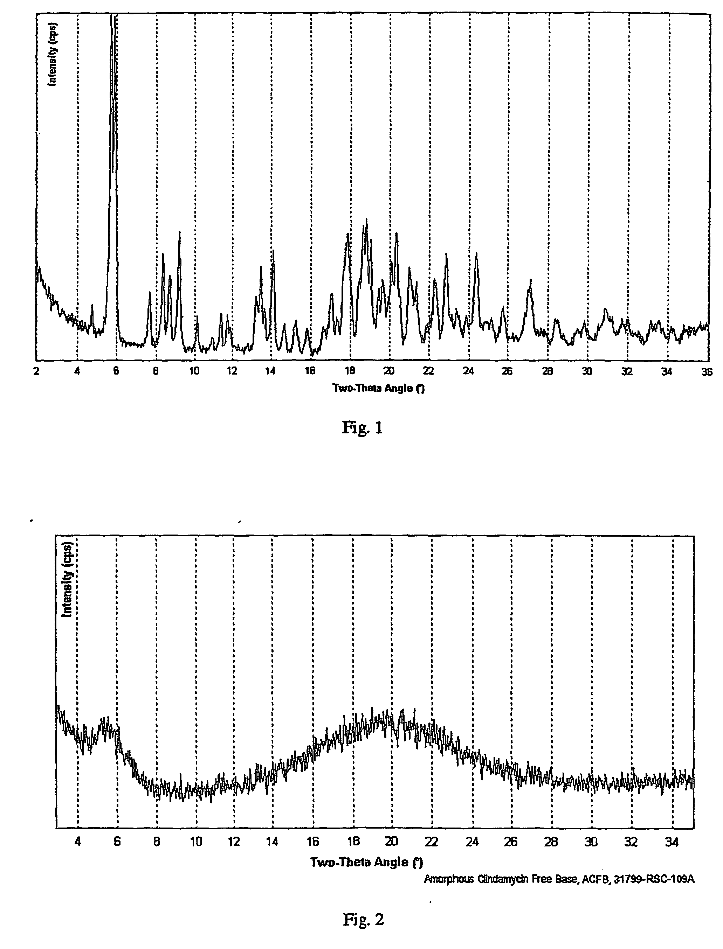

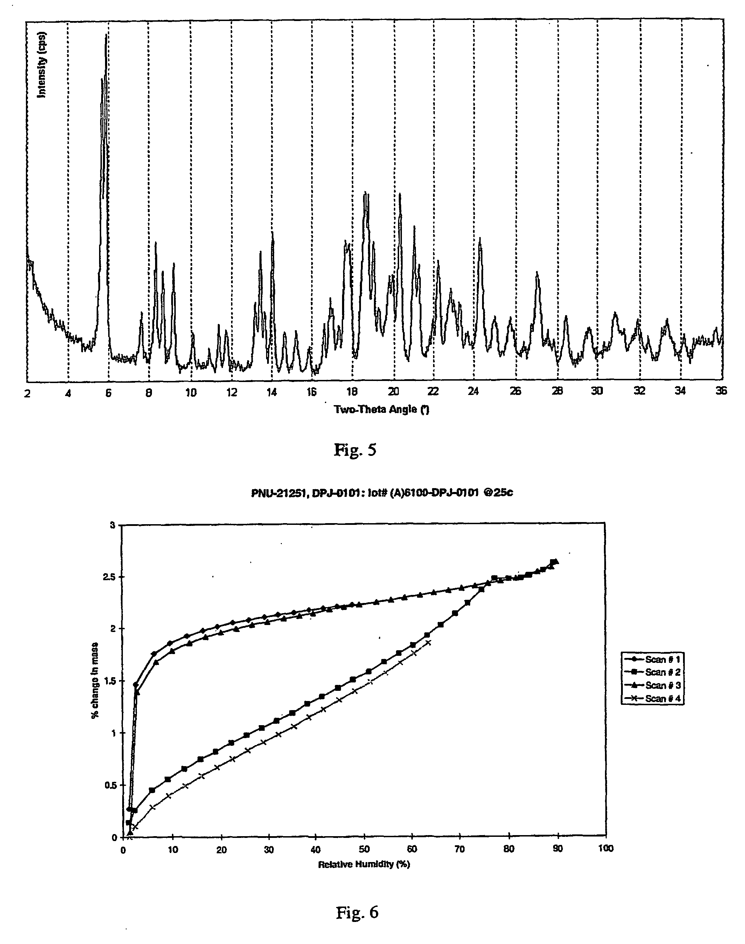

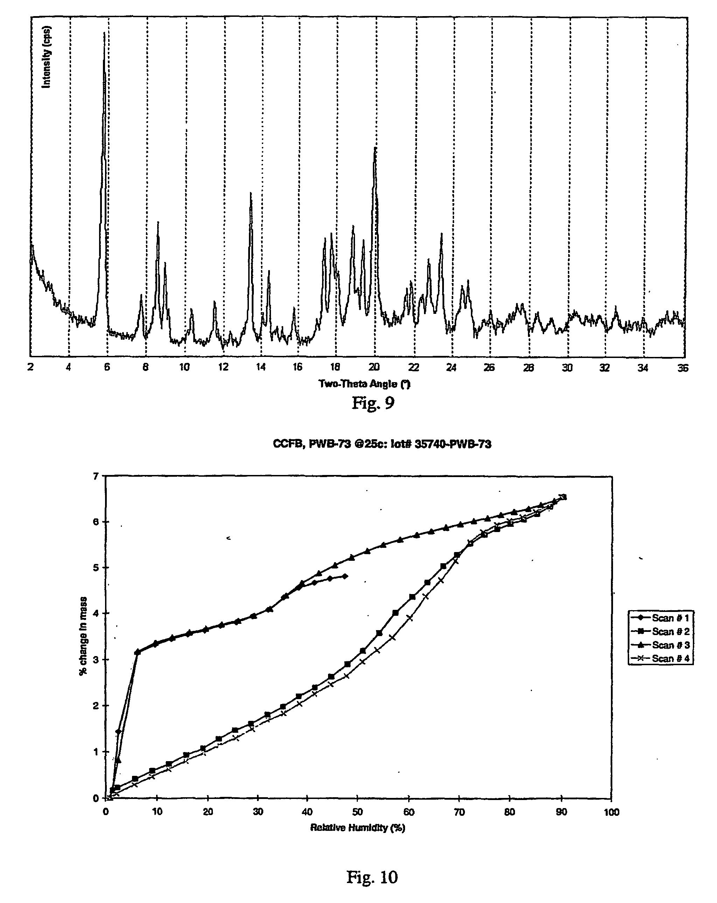

Accordingly, the inventors herein have succeeded in preparing clindamycin free base that is substantially crystalline in nature. The crystalline clindamycin of the present invention can be used as a drug substance in pharmaceutical formulations which can take advantage of the water insolubility of the free base form of clindamycin. Different crystalline polymorphic and pseudopolymorphic forms of clindamycin free base exist of which three are identified herein as Forms I, II and III. The crystalline clindamycin free base Form I is characterized by an X-ray powder diffraction pattern substantially as shown in Figure 1 and by a differential scanning calorimetry exhibiting an endotherm having an extrapolated onset temperature of about 67°C, a peak temperature of about 69°C and an associated heat of about 51 J/g. Form II is characterized by an X-ray powder diffraction pattern substantially as shown in Figure 5 and by a differential scanning calorimetry exhibiting an endotherm having an extrapolated onset temperature of about 63°C, a peak temperature of about 75°C and an associated heat of about 65 J/g. Form III is characterized by an X-ray powder diffraction pattern substantially as shown in Figure 9 and by a differential scanning calorimetry exhibiting an endotherm having an extrapolated onset temperature of about 64°C, a peak temperature of about 69°C and an associated heat of about 69 J/g.

Thus, the present invention, in one embodiment, is directed to a substantially crystalline clindamycin free base. The crystalline clindamycin free base in this and other embodiments herein is preferably of Form I, Form II or Form III. It is preferred that the crystalline material be substantially of one polymorphic or pseudopolymorphic Form such as Form I, Form II, or Form III although combinations of any two or more Forms are within the scope of the present invention. Compositions in which the clindamycin present is crystalline free base of one or more polymorphic or pseudopolymorphic Form in combination with amorphous material are less preferable.

In another aspect, the present invention is directed to a clindamycin free base drug substance comprising, preferably, at least about 10% (w/w) crystalline clindamycin free base, more preferably, at least about 50% (w/w) crystalline clindamycin free base, more preferably, substantially all crystalline clindamycin free base.

The present invention, in another embodiment, is directed to pharmaceutical compositions comprising crystalline clindamycin free base in a pharmaceutically acceptable formulation. The crystalline clindamycin free base is present in the composition at a concentration of, preferably, at least about 1% (w/w), more preferably at least about 10%, more preferably at least about 50% (w/w) and most preferably, at least about 80% (w/w) crystalline clindamycin free base or more.

The crystalline clindamycin free base of the present invention is substantially insoluble in water and for this reason, the material can be incorporated into formulations which exhibit extended or sustained release characteristics. Thus in another embodiment, the present invention is directed to an extended release formulation comprising crystalline clindamycin free base in a pharmaceutically acceptable formulation. The extended release formulation is suitable for administering to a patient at a frequency of, preferably, not more than twice a day and more preferably, not more once a day. The extended release formulation for twice a day and once a day administration provide efficacious blood levels of clindamycin for at least 12 hours and at least 24 hours, respectively. The extended release formulations of clindamycin comprises, preferably, at least about 1% (w/w), at least about 10% (w/w), at least about 50% (w/w), at least about 80% (w/w) or more crystalline clindamycin free base.

In another embodiment, the present invention is directed to a process for preparing crystalline clindamycin free base. The process comprises providing an aqueous solution containing a clindamycin salt; adding an alkali to the solution to form a precipitate; and crystallizing clindamycin free base from the precipitate. Preferably, the clindamycin salt is clindamycin hydrochloride and the alkali is sodium hydroxide.

The present invention in another embodiment is directed to another process for preparing crystalline clindamycin free base. The process comprises: providing an aqueous solution containing a water-soluble organic liquid and an alkalai; adding an aqueous solution of a clindamycin salt to the aqueous solution containing the water-soluble organic liquid; and isolating crystallized clindamycin free base formed therein. The clindamycin salt is preferably clindamycin hydrochloride. The organic liquid is preferably an alcohol, such as methanol or ethanol. The alkali is most preferably sodium hydroxide

Another embodiment of the present invention involves a process for preparing a sustained release clindamycin composition, the process comprising providing an aqueous solution containing a clindamycin salt; crystallizing clindamycin free base upon adding an alkali to the solution; and incorporating the crystallized clindamycin free base into a pharmaceutically acceptable sustained-release formulation. Preferably, at least about 10% (w/w), at least about 50% (w/w) or at least about 80% (w/w) or more crystalline clindamycin free base is incorporated into the formulation

This present invention is also directed to the use of crystalline clindamycin free base for the manufacture of a medicament for treating a medical condition with clindamycin in a patient in need thereof. The method comprises providing a composition containing a sustained release formulation of crystalline clindamycin free base, and administering the composition to the patient. The sustained release formulation comprises at least about 10% (w/w), at least about 50% (w/w), at least about 80% (w/w) or more crystalline clindamycin free base in a pharmaceutically acceptable formulation.

Among the several advantages achieved by the present invention, therefore, may be noted the provision of a novel form of clindamycin free base which is crystalline in nature; the provision of novel polymorphs and pseudopolymorphs of crystalline clindamycin free base; the provision of novel processes for the preparation of the crystalline clindamycin free base; the provision of a new crystalline clindamycin which can be advantageously used in pharmaceutical compositions; the provision of a form of the free base of clindamycin which can be incorporated into new pharmaceutical formulations based upon the water insolubility of clindamycin free base which have different properties than the water-soluble salts of clindamycin; and the provision of novel extended release formulations which are suitable for twice a day or once a day administration based upon the water insolubility of the free base.

### BRIEF DESCRIPTION OF THE DRAWINGS

Figure 1 shows a powder X-ray diffraction profile of crystalline clindamycin free base Form I.

Figure 2 shows a powder X-ray diffraction profile of amorphous clindamycin free base Form I.

Figure 3 shows a moisture sorption profile for crystalline clindamycin free base Form I.

Figure 4 shows a differential scanning calorimetry thermogram of crystalline clindamycin free base Form I.

Figure 5 shows a powder X-ray diffraction profile of crystalline clindamycin free base Form II.

Figure 6 shows a moisture sorption profile for crystalline clindamycin free base Form II.

Figure 7 shows a differential scanning calorimetry thermogram of crystalline clindamycin free base Form II.

Figure 8 compares the powder X-ray diffraction spectra of Form I (top) and Form II (bottom) crystalline clindamycin free base.

Figure 9 shows a powder X-ray diffraction profile of crystalline clindamycin free base Form III.

Figure 10 shows a moisture sorption profile for crystalline clindamycin free base Form III.

Figure 11 shows a differential scanning calorimetry thermogram of crystalline clindamycin free base Form III.

Figure 12 compares the powder X-ray diffraction spectra of Form I (top) and Form III (bottom) crystalline clindamycin free base.

Figure 13 compress the powder X-ray diffraction spectra of phase pure crystalline clindamycin free base Form I (top), a 50% mixture of crystalline Form I and amorphous clindamycin (middle) and phase pure amorphous clindamycin free base (bottom).

### DETAILED DESCRIPTION OF THE INVENTION

The present invention provides a new clindamycin free base material which is substantially crystalline in nature. By substantially crystalline it is meant that at least about 80%, more preferably, at least about 90%, more preferably, at least about 95%, more preferably, at least about 99% to 100% of the material is crystalline on a weight per total weight basis (w/w). Substantially all with respect to a composition containing a clindamycin compound, such as clindamycin free base or crystalline clindamycin free base or a different crystalline form thereof, is intended to mean, preferably at least 95% (w/w), more preferably at least 98% and most preferably, at least 99% to 100% of the composition is comprised of the clindamycin compound. By "about" with respect to a referenced value is intended to include a range of values from 1% below to 1% above the referenced value. For example, "about" 1.0 is intended to include a range of values from 0.99 to 1.01.

Crystallinity refers to the ordered arrangement of molecules within a solid. The molecules in a crystal are arranged in fixed geometric patterns or lattices. By way of contrast, solids which are in an amorphous state are comprised of molecules which are unoriented and arranged in short range order. The percent crystallinity of clindamycin free base is intended to reference the amount of crystalline material to the total of crystalline and amorphous free base present in a composition. Assessing crystallinity and determining percent crystallinity can be performed by any of a number of methods known in the art. Such methods include x-ray diffraction, such as using powder x-ray diffraction to look for sharp peaks characteristic of crystallinity, inspection for bright birefringence using a polarized microscope, and thermal analysis methods such as, differential scanning calorimetry.

The crystalline clindamycin free base can be in different crystalline forms, i.e polymorphs and/or pseudopolymorphs or solvates. The different forms of crystalline clindamycin free base can be distinguished by methods known in the art such as in the differences in x-ray diffraction patterns and in melting points. X-ray powder diffraction analysis can be performed according to methods known in the art (see for example, Buckton et al, *Int. J. Pharm. 179*:141-58; Giacovazzo, C. et al., in *Fundamentals of Crystallography*, Oxford University Press; 1996; Jenkins et al, in *Introduction to X-Ray Powder Diffractometry*, John Wiley & Sons, New York, 1996). Crystalline clindamycin free base can be distinguished from amorphous material by the presence of peaks in the x-ray diffraction pattern and different polymorphs or pseudopolymorphs can be characterized and distinguished by the d-spacing in Angstroms and intensity of peaks in the x-ray diffraction pattern. Typically, material that is substantially all crystalline will give rise to sharp, high frequency peaks having narrow widths limited by instrumental resolution. Materials having lesser percent crystallinity give rise to broader, more diffuse diffraction peaks. Amorphous material gives a broad, very low frequency halo with an occasional harmonic.

Differential scanning calorimetry (DSC) can also characterize crystalline materials (see Hohne et al, *Differential Scanning* Calorimetry, Springer, Berlin, 1996). DSC can identify an endotherm with an extrapolated onset temperature, a peak temperature and an associated heat in Joules/gram (J/g) which represents a melting point transition for the particular polymorph or pseudopolymorph.

Crystalline clindamycin free base is believed to have a number of polymorphs/pseudopolymorphs and three have been identified herein. The three polymorphs/pseudopolymorphs referenced herein as Forms I, II and III, give sharp, narrow x-ray diffraction peaks and DSC endotherms. The distinctive x-ray diffraction patterns are as show in Figures 1,5 and 9. The DSC endotherms show an extrapolated onset temperature of about 66.9°C, a peak temperature of about 69. 1°C and an associated heat of about 50.7 J/g for Form I. The DSC endotherm for Form II has an extrapolated onset temperature of about 62.7°C, a peak temperature of about 75.1°C and an associated heat of about 65.0 J/g. The DSC endotherm for Form III has an extrapolated onset temperature of about 64.4°C, a peak temperature of about 69.4°C and an associated heat of about 69.4 J/g.

### Clindamycin drug substance of the invention

The crystalline clindamycin free base of the present invention comprises clindamycin drug substances made up of crystalline, microcrystalline or nanocrystalline, clindamycin free base. A "drug substance" as referenced herein is intended to mean a material which can be used in the diagnosis, cure, mitigation, treatment or prevention of disease. The crystalline clindamycin free base drug substance of the present invention is preferably, substantially all crystalline in nature. It is generally the case that crystallinity and the polymorphic forms of crystalline drug substances play a major role in affecting drug dissolution, chemical stability and drug bioabioavailability. Thus, it is preferred that the crystalline material be substantially of one crystalline form such as Form I, Form II, or Form III; although, combinations of any two or more such forms are, also , within the scope of the present invention. Crystalline clindamycin drug substances of the present invention in which the clindamycin is present as crystalline free base in combination with amorphous free base are less preferable but still within the scope of the present invention. Thus, preferably, from about 1% (w/w) to about 100% (w/w), more preferably, from about 10% (w/w) to about 100% (w/w), more preferably from about 25% (w/w) to about 100% (w/w), more preferably from about 60% (w/w) to about 100% (w/w), more preferably from about 80% (w/w) to about 100% (w/w), more preferably from about 90% (w/w) to about 100% (w/w), and most preferably from about 95% (w/w) to about 100% (w/w) of the clindamycin in a clindamycin drug substance of the invention is crystalline free base. The crystalline free base in the crystalline clindymycin free base drug substance is preferably comprised of at least 80% (w/w) (w/w), more preferably at least 90% (w/w), and still more preferably at least 95% (w/w) one crystalline form such as Form I, Form II or Form III. In a particular embodiment, substantially all of the clindamycin is crystalline free base, *i*.*e*., the clindamycin drug substance is substantially phase pure. Preferably substantially all of the crystalline for is of one crystalline form such as Form I, Form II or Form III.

It is believed that the crystalline clindamycin free base drug substances and compositions thereof will exhibit greater stability than amorphous clindamycin free base materials and compositions thereof. The term "stability" as used herein is intended to include chemical stability and physical stability. By chemical stability it is meant that under defined storage conditions no substantial degree of chemical degradation or decomposition will occur. By physical stability, it is meant that under defined storage conditions no substantial degree of solid state transformation will occur such as, for example, crystallization, change in crystal form, conversion to amorphous material, hydration, dehydration, aggregation, solvation and the like.

Crystalline clindamycin free base or a crystalline clindamycin drug substance of the invention can be prepared by any suitable process, not limited to processes described herein.

One illustrative process of preparing crystalline clindamycin free base involves forming the amorphous free base as a precipitate in aqueous medium followed by agitation to crystallize the free base from the precipitate. An illustrative example of the method involves first dissolving a salt of clindamycin, e.g., clindamycin hydrochloride in a solvent, preferably a polar solvent such as, for example, water. This if followed by adding an alkali material, i.e. a base, in an aqueous vehicle such as for example, a NaOH solution, such as, for example, preferably from about 0.01 to about 10 N NaOH solution, more preferably from about 0.1 to about 1 N NaOH, and more preferably about 0.5 N NaOH. This results in precipitation of the amorphous free base. The amorphous free base is then crystallized by agitation of the precipitate by, for example, by sonicating or manually shaking the precipitate, or by both sonicating and manually shaking the precipitate suspended in the aqueous medium. The crystallized free base is then preferably harvested by centrifugation, followed by removal of the liquid portion. The crystallized free base is preferably washed in at least one washing step involving adding a wash solution, sonicating, shaking, centrifuging and removing the wash solution from the crystalline materia. The wash solution is preferably aqueous, more preferably water.

In an alternate method, crystalline clindamycin free base can be produced by a slow addition of a clindamycin salt, such as clindamycin hydrochloride, dissolved in a polar solvent such as water to an aqueous alkaline solution containing a water-soluble organic substance, preferably an alcohol co-solvent. The aqueous solution containing an alkali with an alcohol co-solvent is prepared by adding the alkali, i.e. base, in an aqueous vehicle such as, for example, a NaOH solution. The NaOH solution can be, for example, preferably from about 0.01 to about 10 N NaOH solution, more preferably from about 0.1 to about 1 N NaOH, and more preferably about 0.5 N NaOH. The alcohol co-solvent is present, preferably in an amount of from about 2% to about 20%, more preferably from about 5% to about 10%. Any of a number of alcohols that are readily miscible with water can be used, preferably, methanol, ethanol, *n*-propanol, *t*-butanol and the like. Typically alcohols of higher molecular weight are less soluble in water and less preferred. Diols such as 1,2, ethanediol (ethylene glycol), 1,2 propanediol (propylene glycol) and 1,2 butanediol and triols such as 1,2,3 propantriol (glycerol) and the like can also be used as co-solvent. It is also possible to use an aqueous solution of a water-soluble organic substance such as, for example, sodium acetate.

An aqueous solution of a clindamycin salt, such as, for example clindamycin hydrochloride is prepared and slowly added to the alkali solution with alcohol co-solvent, preferably over a period of from about 15 minutes to about 4 hours, more preferably from about 30 minutes to about 2 hours and most preferably from about 45 minutes to 75 minutes. Crystallization is allowed to proceed for 1 to 24 hours and the crystalline free base material is isolated by filtration, centrifugation and decanting or the like. In a preferred variation of this method, the clindamycin hydrochloride solution is added in a multi-phase infusion schedule such as, for example, a first phase of slow infusion over about one hour, followed by a faster infusion phase over about 30 min and concluding with slow infusion phase over about one hour.

The material obtained by either of the methods above is isolated and dried, for example, under a stream of humidified nitrogen. The dry material can be further processed such as by grinding to produce a dry powder. Preferably, the substantially pure salt of clindamycin has a purity of more than 95% (w/w) such as, for example, 99% (w/w) or greater.

A clindamycin drug substance or drug powder prepared according to the above process or any other process can be administered orally, rectally or parenterally without further formulation, or in a simple suspension in water or another pharmaceutically acceptable liquid. Alternatively, the clindamycin drug substance or drug powder can be directly filled into capsules for oral administration. Preferably, however, the clindamycin drug substance or drug powder is subjected to further processing, typically with one or more excipients, to prepare a pharmaceutical composition, for example an oral modified release dosage form or an intraorally interacting dosage form, as described hereinbelow.

### Pharmaceutical compositions

Crystalline clindamycin free base or a crystalline clindamycin drug substance as provided herein can be further formulated together with one or more pharmaceutically acceptable excipients to produce a pharmaceutical composition. The term "excipient" herein means any substance, not itself a therapeutic agent, used as a carrier or vehicle for delivery of a therapeutic agent to a subject or added to a pharmaceutical composition to improve its handling, drug release, or storage properties or to permit or facilitate formation of a dose unit of the composition into a discrete article such as a capsule or tablet suitable for oral administration. Excipients include, by way of illustration and not limitation, diluents, disintegrants, binding agents, adhesives, wetting agents, lubricants, glidants, crystallization inhibitors, release modifying agents, plasticizers, surface modifying agents, substances added to mask or counteract a disagreeable taste or odor, flavors, dyes, fragrances, and substances added to improve appearance of the composition.

The pharmaceutical composition of the present invention preferably contains an antimicrobially effective amount of crystalline clindamycin free base for the treatment of an antibiotic infection in a subject. The amount of crystalline clindamycin free base appropriate for any given subject depends upon the species of the subject, the weight of the subject, and the dosage equivalency of the particular lincosamide to be administered to the subject. When the subject is a human adult, the pharmaceutical composition preferably contains about 10 mg to about 800 mg, more preferably about 25 mg to about 300 mg, even more preferably about 50 mg to about 200 mg, and most preferably about 50 mg to about 150 mg of crystalline clindamycin free base.

The pharmaceutical compositions of the present invention preferably comprise at least about 1% (w/w), more preferably at least about 10% (w/w), more preferably at least about 50% (w/w), even more preferably at least about 80% (w/w) crystalline clindamycin free base.

Excipients employed in compositions of the invention can be solids, semi-solids, liquids or combinations thereof. Compositions of the invention containing excipients can be prepared by any known technique of pharmacy that comprises admixing an excipient with a drug or therapeutic agent. A composition of the invention contains a desired amount of clindamycin per dose unit and, if intended for oral administration, can be in the form, for example, of a tablet, a caplet, a pill, a hard or soft capsule, a lozenge, a cachet, a dispensable powder, granules, beads, a suspension, an elixir, a liquid, or any other form reasonably adapted for such administration. If intended for parenteral administration, it can be in the form, for example, of a suspension. If intended for rectal administration, it can be in the form, for example, of a suppository. Presently preferred oral dosage forms that are discrete dose units each containing a predetermined amount of the drug, such as tablets or capsules. Presently more preferred are oral modified release dosage forms including, but not limited to coated tablets, matrix tablets, coated beads, matarix beads and capsule formulations.

Non-limiting examples of excipients that can be used to prepare pharmaceutical compositions of the invention are as follows.

Compositions of the invention optionally comprise one or more pharmaceutically acceptable diluents as excipients. Suitable diluents illustratively include, either individually or in combination, lactose, including anhydrous lactose and lactose monohydrate; starches, including directly compressible starch and hydrolyzed starches (*e*.*g*., Celutab™ and Emdex™); mannitol; sorbitol; xylitol; dextrose *(e.g.,* Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of α- and amorphous cellulose *(e.g.,* Rexcel™) and powdered cellulose; calcium carbonate; glycine; bentonite; polyvinylpyrrolidone; and the like. Such diluents, if present, constitute in total about 5% to about 99%, preferably about 10% to about 85%, and more preferably about 20% to about 80%, of the total weight of the composition. The diluent or diluents selected preferably exhibit suitable flow properties and, where tablets are desired, compressibility.

Compositions of the invention optionally comprise one or more pharmaceutically acceptable disintegrants as excipients, particularly for tablet formulations. Suitable disintegrants include, either individually or in combination, starches, including sodium starch glycolate (*e*.*g*., Explotab™ of PenWest) and pregelatinized corn starches (*e.g.*, National™ 1551, National™ 1550, and Colorcon™ 1500), clays (*e*.*g*., Veegum™ HV), celluloses such as purified cellulose, microcrystalline cellulose, methylcellulose, carboxymethylcellulose and sodium carboxymethylcellulose, croscarmellose sodium *(e.g.,* Ac-Di-Sol™ of FMC), alginates, crospovidone, and gums such as agar, guar, locust bean, karaya, pectin and tragacanth gums.

Disintegrants may, if desired, be added at any suitable step during the preparation of the composition, particularly prior to granulation or during a lubrication step prior to compression. Such disintegrants, if present, constitute in total about 0.2% to about 30%, preferably about 0.2% to about 10%, and more preferably about 0.2% to about 5%, of the total weight of the composition.

Compositions of the invention optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, either individually or in combination, acacia; tragacanth; sucrose; gelatin; glucose; starches such as, but not limited to, pregelatinized starches (*e*.*g*., National™ 1511 and National™ 1500); celluloses such as, but not limited to, methylcellulose and carmellose sodium *(e.g.,* Tylose™); alginic acid and salts of alginic acid; magnesium aluminum silicate; PEG; guar gum; polysaccharide acids; bentonites; povidone, for example povidone K-15, K-30 and K-29/32; polymethacrylates; HPMC; hydroxypropylcellulose *(e.g.,* Klucel™); and ethylcellulose *(e.g.,* Ethocel™). Such binding agents and/or adhesives, if present, constitute in total about 0.5% to about 25%, preferably about 0.75% to about 15%, and more preferably about 1% to about 10%, of the total weight of the composition.

Compositions of the invention optionally comprise one or more pharmaceutically acceptable wetting agents as excipients. Such wetting agents are preferably selected to maintain the clindamycin in close association with water, a condition that is believed to improve bioavailability of the composition.

Non-limiting examples of surfactants that can be used as wetting agents in compositions of the invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides *(e.g.,* Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e*.*g*., Tween™ 80 of ICI), propylene glycol fatty acid esters, for example propylene glycol laurate *(e.g.,* Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, preferably about 0.4% to about 10%, and more preferably about 0.5% to about 5%, of the total weight of the composition.

Compositions of the invention herein can comprise a modified release formulation. A modified release formulation or modified release dosage form refers to a preparation in which the rate and/or location of release of the active drug substance is different from that of the conventional dosage form administered by the same route (for review, see Shargel and Yu in *Applied Biopharmaceutics & Pharmacokinetics, Fourth Edition,* Mehalik, Ed, Appleton & Lange, Stamford, CT, 1999, pp. 169-203). Modified release dosage forms include extended-release dosage forms, delayed-release dosage forms and targeted-release dosage forms. Extended-release dosage forms include dosage forms which have been referred to as extended-release-rate dosage forms, sustained release dosage forms, sustained action dosage forms, prolonged action dosage forms, long action dosage forms or retarded release dosage forms. Such extended release dosage forms allow a reduction in dosage frequency as compared to an immediate release, conventional dosage form of the same drug. For example, an extended-release dosage form which allows the formulated drug to be admininstered twice a day, i.e. every 12 hours compared to a conventional dosage form that is administered four times a day, i.e. every 6 hours, or three times a day, i.e. every 8 hours, constitutes an extended release dosage form. Delayed-release dosage forms release at least a portion of the drug at a time or at times other than promptly after administration, although another portion may be released promptly after administration. Enteric coated tables are one commonly known delayed-release product. Targeted-release dossage forms release drug at or near the intended physiologic site of action.

One approach for obtaining a prolonged action of a drug is to select a form of the drug which has reduced solubility such that drug dissolution occurs slowly over a period of several hours. Thus, the free base of clindamycin, which has a low water solubility, provides a form of clindamycin which would be expected to have slow dissolution and prolonged drug action as compared to the water soluble hydrochloride salt of clindamycin. Further formulation of the clindamycin free base can be used to produce an extended-release preparation. Such extended-release preparations include, but are not limited to, pellet-form sustained release preparations, in which pre-formed cores are coated with the drug which is further overlayed with a protective coating to allow sustained or prolonged release of the drug; prolonged-action tablets, in which a slow dissolution formulaton of the drug is incorporated into a tablet; ion exchange preparations in which a nonabsorbable complex is formed with the drug and an ion exchange resin an drug is released by exchange with ions in the gastrointestinal tract; core tablet preparations in which the core of the tablet is a slow drug-release component and the outer portion of the tablet is a rapid-release formulation of the drug; gum-form matrix tablets in which an excipient in the tablet swells in the presence of water to produce a matrix slowing drug dissolution; microencapsulation in which drug particles are coated with a material which results in a gradual release of drug from a tablet; polymeric matrix tablets in which a polymer matrix provides a slow and constant drug release; and osmotic controlled release preparations in which drug delivery is controlled by an osmotic ally driven release of the drug and the like (for review, see Shargel and Yu, *supra*, 1999).

In certain embodiments, the pharmaceutical compositions of the present invention include one or more pharmaceutically acceptable release modifying agents which can serve as a matrix for the crystalline clindamycin free base drug substance to produce a slow and continuous drug release. Such release modifying agents include gum-form materials such as methylcellulose, gum taragacanth, Veegum and alginic acid. Other release modifying agents include polymeric substances that can form a matrix including synthetic, semisynthetic or natural polymers including but not limited to ethyl cellulose, hydroxypropylmethyl cellulose phthalate, cellulose acetate phthalate, cellulose, acetate trimellitate, polymethacrylates, hydroxypropylmethylcellulose, hydroxyethyl cellulose, hydroxypropyl cellulose,methylcellulose, carboxymethylcellulose, polyethylene glycol, polyvinylpyrrolidone (PVP), polyvinyl alcohol, cellulose acetate phthalate, ammonium methacrylate copolymer and methacrylic acid copolymer, hydroxypropylmethylcellulose phthalate, cellulose acetate phthalate, cellulose acetate trimellitate, polymethacrylates or mixtures thereof. Further release modifying agents include lipids such as, alkanes, mono- di- or tri glycerides of fatty acids, free fatty acids, fatty alcohols, fixed oils, fats and waxes, such as carnauba wax and bees wax.

Although crystalline clindamycin free base could potentially provide a basis for preparation of formulations with prolonged action, an immediate release formulation of the free base would be less preferred for achieving prolonged drug action. This is because an immediate release formulation of clindamycin free base is likely to release a significant portion of the drug in the stomach. Secretion of HCl in the stomach results in a low pH of about 1 to 2 in the presence of food and a fasting pH of about 3 to 5. At low pH conditions, the available chloride counterion is expected to convert the immediate released clindamycin free base into the water-soluble hydrochloride salt. This would, however, have the result of substantially increasing the dissolution rate to that approaching the hydrochloride salt and little or no prolonged action of the drug as compared to the same clindamycin formulation prepared with the hydrochloride salt, would be expected. For this reason, in certain embodiments, it is desirable to provide a delayed release formulation of the free base such as an enteric coating of the preparation or to incorporate buffering agents into the composition.

Enteric coating polymers that can be used with the present invention include cellulose acetate phthalate, hydroxypropyl methylcellulose phthalate, hydroxypropylmethyl cellulose acetate succinate, polyvinyl acetate phthalate, carboxymethylethyl cellulose, coolymerized methacrylic acid/methacrylic acid methyl esters. (see U.S. Pat. Nos. 6224911 and 4,857,337 for a description of enteric coatings and methods for applying such coatings).

Incorporation of a buffering agent into the formulation can also diminish the conversion of clindamycin free base into the hydrochloride salt in embodiments in which a prolonged drug action is desired. Buffering agents can be any of a wide variety of agents including, but are not limited to sodium, potassium, calcium or magnesium carbonate or bicarbonates; calcium, magnesium or aluminum hydroxide; magnesium oxide; sodium, potassium, calcium or magnesium salts of citric acid, fumaric acid, adipic acid, tartaric acid, ascorbic acid, glutamic acid or aspartic acid and the like.

Compositions of the invention optionally comprise one or more pharmaceutically acceptable plasticizers. Suitable plasticizers include either individually or in combination, dibutylsebacate, propylene glycol, triethylcitrate, tributylcitrate, castor oil, acetylated monoglycerides, acetyl triethylcitrate, acetyl butylcitrate, diethyl phthalate, dibutyl phthalate, triacetin, fractionated coconut oil (medium-chain triglycerides).

Compositions of the invention optionally comprise one or more pharmaceutically acceptable lubricants (including anti-adherents and/or glidants) as excipients. Suitable lubricants include, either individually or in combination, glyceryl behapate (*e*.*g*., Compritol™ 888); stearic acid and salts thereof, including magnesium, calcium and sodium stearates; hydrogenated vegetable oils *(e.g.,* Sterotex™); colloidal silica; talc; waxes; boric acid; sodium benzoate; sodium acetate; sodium fumarate; sodium chloride; DL-leucine; PEG (*e*.*g*., Carbowax™ 4000 and Carbowax™ 6000); sodium oleate; sodium lauryl sulfate; and magnesium lauryl sulfate. Such lubricants, if present, constitute in total about 0.1 % to about 10%, preferably about 0.2% to about 8%, and more preferably about 0.25% to about 5%, of the total weight of the composition.

Magnesium stearate is a preferred lubricant used, for example, to reduce friction between the equipment and granulated mixture during compression of tablet formulations.

Suitable anti-adherents include talc, cornstarch, DL-leucine, sodium lauryl sulfate and metallic stearates. Talc is a preferred anti-adherent or glidant used, for example, to reduce formulation sticking to equipment surfaces and also to reduce static electricity in the blend. Talc, if present, constitutes about 0.1% to about 10%, more preferably about 0.25% to about 5%, and still more preferably about 0.5% to about 2%, of the total weight of the composition.

Glidants can be used to promote powder flow of a solid formulation. Suitable glidants include colloidal silicon dioxide, starch, talc, tribasic calcium phosphate, powdered cellulose and magnesium trisilicate. Colloidal silicon dioxide is particularly preferred.

Other excipients such as colorants, flavors and sweeteners are known in the pharmaceutical art and can be used in compositions of the present invention. Tablets can be coated, for example with an enteric coating, or uncoated. Compositions of the invention can further comprise, for example, buffering agents.

Solid dosage forms of the invention can be prepared by any suitable process, not limited to processes described herein.

An illustrative process comprises (a) a step of blending crystalline clindamycin free base or a crystalline clindamycin drug substance of the invention with one or more excipients to form a blend, and (b) a step of tableting or encapsulating the blend to form tablets or capsules respectively.

In a another illustrative process, the manufacture of a modified release dosage form, comprises, (a) a step of blending crystalline clindamycin free base or a crystalline clindamycin drug substance of the invention with one or more release modifying agents and one or more other excipients to form a blend, (b) an optional step of granulating said blend (c) an optional step of drying (d) an optional step of blending with one or more excipients and (e) a step of tableting the blend to form tablets.

In a another illustrative process, the manufacture of a modified release dosage form, comprises, (a) a step of forming small spheres of crystalline clindamycin free base or a crystalline clindamycin drug substance of the invention and one or more other excipients to form a blend, (b) a step of coating said spheres with one or more layers comprising release modifying substances(d) an optional step of tableting or encapsulating the coated spheres to form tablets or capsules respectively.

In a another illustrative process, the manufacture of an intraorally interacting dosage form, comprises, (a) an optional step of coating particles of crystalline clindamycin free base or a crystalline clindamycin drug substance with one or more excipients in order to mask the taste as known by those skilled in the art, (b) a step of blending said particles with suitable excipients, e.g., flavors and sweeteners.

### Clindamycin dosage

Clindamycin dosage forms of the invention preferably comprise clindamycin in a daily dosage amount of about 10 mg to about 3 g, more preferably about 20 mg to about 2 g, and most preferably about 500 mg to about 1.8 g.

Compositions of the invention comprise one or more orally deliverable dose units. Each dose unit comprises clindamycin in a therapeutically effective amount that is preferably about 5 mg to about 1000 mg, for example from about 20 to about 600 mg. The term "dose unit" herein means a portion of a pharmaceutical composition that contains an amount of a therapeutic or prophylactic agent, in the present case clindamycin, suitable for a single oral administration to provide a therapeutic effect. Typically one dose unit, or a small plurality (up to about 4) of dose units, in a single administration provides a dose comprising a sufficient amount of the agent to result in the desired effect. Administration of such doses can be repeated as required, typically at a dosage frequency of up to about 4 times per day. Extended release formulations would be administered at a dosage frequency of no more than twice a day or once a day.

It will be understood that a therapeutically effective amount of clindamycin for a subject is dependent *inter alia* on the body weight of the subject. A "subject" herein to which a therapeutic agent or composition thereof can be administered includes a human patient of either sex and of any age, and also includes any nonhuman animal, particularly a warm-blooded animal, more particularly a domestic or companion animal, illustratively a cat, dog or horse. When the subject is a child or a small animal (*e*.*g*., a dog), for example, an amount of clindamycin relatively low in the preferred range of about 10 mg to about 1000 mg is likely to provide blood concentrations consistent with therapeutic effectiveness. Where the subject is an adult serum human or a large animal (*e.g.*, a horse), achievement of such blood serum concentrations of clindamycin are likely to require dose units containing a relatively greater amount of clindamycin.

Typical dose units in a composition of the invention contain about 10, about 20, about 25, about 37.5, about 50, about 75, about 100, about 125, about 150, about 175, about 200, about 250, about 300, about 350, about 400, about 500, about 600, about 700, about 800, about 900 and about 1000 mg of clindamycin. For an adult human, a therapeutically effective amount of clindamycin per dose unit in a composition of the present invention is typically about 100 mg to about 1000 mg. The preferred dose for an adult is preferably 200-700 mg, and more preferably, about 300 or about 600 mg. A dose unit containing a particular amount of clindamycin can be selected to accommodate any desired frequency of administration used to achieve a desired daily dosage. The daily dosage and frequency of administration, and therefore the selection of appropriate dose unit, depends on a variety of factors, including the age, weight, sex and medical condition of the subject, and the nature and severity of the condition or disorder, and thus may vary widely.

The term "oral administration" herein includes any form of delivery of a therapeutic agent or a composition thereof to a subject wherein the agent or composition is placed in the mouth of the subject, whether or not the agent or composition is immediately swallowed. Thus "oral administration" includes buccal and sublingual as well as esophageal administration. Absorption of the agent can occur in any part or parts of the gastrointestinal tract including the mouth, esophagus, stomach, duodenum, ileum and colon. The term "orally deliverable" herein means suitable for oral administration.

### Method of treatment

The present invention is further directed to a therapeutic method of treating a bacterial infection where treatment with a clindamycin is indicated, the method comprising oral administration of a composition of the invention to a subject in need thereof. The dosage regimen to prevent or cure the infection preferably corresponds to 150 to 450 mg every 6 hours for adults and for extended-release formulations, 300 to 900 mg every 12 hours or 600 to 1800 mg every 24 hours. Pediatric patients are typically given 8 to 20 mg/kg/day divided into three or four equal doses. The dosage regimen can be modified in accordance with a variety of factors. These include the Form, age, weight, sex, diet and medical condition of the subject and the nature and severity of the disorder. Thus, the dosage regimen actually employed can vary widely and can therefore deviate from the preferred dosage regimens set forth above.

The compound to be administered in combination with clindamycin can be formulated separately from the clindamycin or co-formulated with the clindamycin in a composition of the invention.

### Industrial Applicability of the invention

Compositions of the invention are useful in treatment and prevention of a wide range of bacterial infections. Such compositions can be used for the treatment of serious infections caused by susceptible anaerobic bacteria, streptococci, pneumococci, and staphylococci, for example serious respiratory tract infections such as empyema, anaerobic pneumonitis and lung abscess; serious skin and soft tissue infections; septicemia; intra-abdominal infections such as peritonitis and intra-abdominal abscess (typically resulting from anaerobic organisms resident in the normal gastrointestinal tract); infections of the female pelvis and genital tract such as endometritis, nongonococcal tubo-ovarian abscess, pelvic cellulitis and postsurgical vaginal cuff infection

Besides being useful for human treatment, compositions of the invention are useful for veterinary treatment of companion animals, exotic animals, farm animals, and the like, particularly mammals.

The following Examples are provided for illustrative purposes only and are not to be interpreted as limiting the scope of the present invention. The Examples will permit better understanding of the invention and better perception of its advantages.

### EXAMPLE 1

This example illustrates a method of preparation of crystalline clindamycin free base, Form I upon addition of NaOH to a solution of clindamycin hydrochloride solution.

Crystalline clindamycin free base, Form I, was generated by lab run (45g scale) as follows:

A clindamycin hydrochloride solution was prepared by dissolving 57.12 gram of clindamycin hydrocholoride in 175 ml of deionized water in a 500 ml beaker. About 130 ml of 1.0 N NaOH was slowly added to the solution. The solution became cloudy and large white sticky ball-like lumps formed at the bottom of the beaker. The solution mixture in the beaker from the preceding step was shaken and sonicated and the ball-like lumps were manually deaggregated.

After deaggregation, the ball-like lumps became small white solid species and were precipitated in the solution. The mixture was then shaken for 10 minutes and sonicated for about 30 minutes. Thereafter, the solution mixture was stirred at moderate speed on a magnetic stirring plate overnight

After overnight stirring, the white precipitated solids in the mixture were uniformly small and cubic-like. Under a polarized microscope the solids were determined to be crystalline.

The mixture was centrifuged at 3000 RPM for 10 minutes and the liquid portion was removed.

The solids remaining in the beaker were washed by adding 50 ml deionized water and 10 ml of 0.5 N NaOH; after shaking and sonicating the mixture was centrifuged at 3000 RPM for 10 minutes.

The liquid portion was removed, and 50 ml of deionized water was added to the beaker. The mixture was then shaken, sonicated and centrifuged. The centrifuged material was filtered with a vacuum filtration facility and the solid portion of the centrifuged mixture remained on the filter while the liquid portion was discharged.

The white solid on the filter was washed five times, each with 50ml of deionized water. Each washing step including shaking, agitation with a glass rod.

After all liquid was discharged, the white solid was removed from filter. The resulting white crystalline material was dried under a gentle stream of nitrogen.

The yield after drying was 44.08 g of crystalline clindamycin free base, which was classified as Form I as described below.

The crystallinity was verified by powder X-ray diffraction.

### EXAMPLE 2

This example illustrates the powder x-ray diffraction analysis of crystalline clindamycin free base, Form I.

Powder X-ray diffraction (PXRD) analysis was used to determine the relative crystallinity of clindamycin as prepared in Example 1 and in subsequent examples below. PXRD data were collected using a Scintag Advanced Diffraction System operating under Scintag's DMS/NT software. This system uses a peltier cooled solid state detector and a copper X-ray source maintained at 45 kV and 40 mA to provide CuKα₁ emission at 1.5406 Å. The beam aperture was controlled using tube divergence and anti-scatter slits of 2 and 4 mm respectively, while the detector anti-scatter and receiving slits were set at 0.5 and 0.3 mm respectively. Data were collected from 2° to 40° two-theta (2θ) angle using a scan step of 0.03°/point and a one second/point integration time. The samples were prepared using Scintag round top-loading stainless steel sample cups, and were fitted with 12 mm diameter aluminum inserts to accommodate small sample volumes.

Crystalline organic compounds usually consist of a large number of atoms, which are arranged in a periodic array in three-dimensional space. Powder X-ray diffraction (PXRD) is considered to be one of the most sensitive methods to determine the crystallinity of solid materials. When excited by x-ray radiation, crystals yield explicit maxima at specific diffraction angles to reflect the interplanar spacings of crystal lattice, as predicted by the Bragg's law. Thus, the PXRD profile of a crystaline material features characteristically sharp, distinct peaks. On the contrary, amorphous or non-crystalline solids typically yield a featureless PXRD profile with broad, diffuse, halos; because, such solids lack the long range order of a repeating crystal lattice.

For comparative purposes, amorphous material was prepared by dissolving Form I crystalline clindamycin free base in methanol and evaporating to dryness to yield a glassy amorphous material.

The crystallinity differences between the amorphous and crystalline clindamycin free base are clearly illustrated by the PXRD analyses as shown in Figs. 1-2. The broad diffused halos without characteristic peak in Figure 2 are indicative of amorphous material for the amorphous clindamycin free base. The clindamycin in this sample was substantially phase pure amorphous clindamycin.

In Figure 1, the appearance of multiple strong and sharp diffraction peaks indicates excellent crystallinity of the crystalline clindamycin free base, which is identified as Form I crystalline clindamycin free base. There is no indication that any amorphous material was present in the crystalline sample such that if any was present, it would have been no more than a minor component.

### EXAMPLE 3

This example illustrates the moisture sorption gravimetry (DMSG) of crystalline clindamycin free base, Form I.

We used DMSG to study moisture sorption and desorption of clindamycin free base. Moisture sorption is an important characteristic of the solid material as any drug molecule might have different moisture sorption profiles in different solid phases. Therefore we have supplemented the DMSG measurements with powder PXRD analysis in this study.

In order to understand the moisture uptake of clindamycin free base and its stability at various humidities was studied using an isothermally controlled atmospheric microbalance (CAM). Approximately 10 mg samples were used in the balance, samples were run as received. The humidity was set at ambient conditions on the day analysis began. Unless specified otherwise, the normal DMSG analysis consisted of three scans: ambient to 90% RH, 90% RH to 0% RH, 0% RH to 90% RH. The scan rate was 3 % RH/step. The mass was measured every two minutes and an equilibrium window of 5 points within 0.001 mg. The RH was changed to the next value (+/- 3%RH) when the mass of the sample was stable to within 0.001 mg. The Visual Basic program dmsgscn2.exe was used to control the data collection and export the information to an Excel spreadsheet.

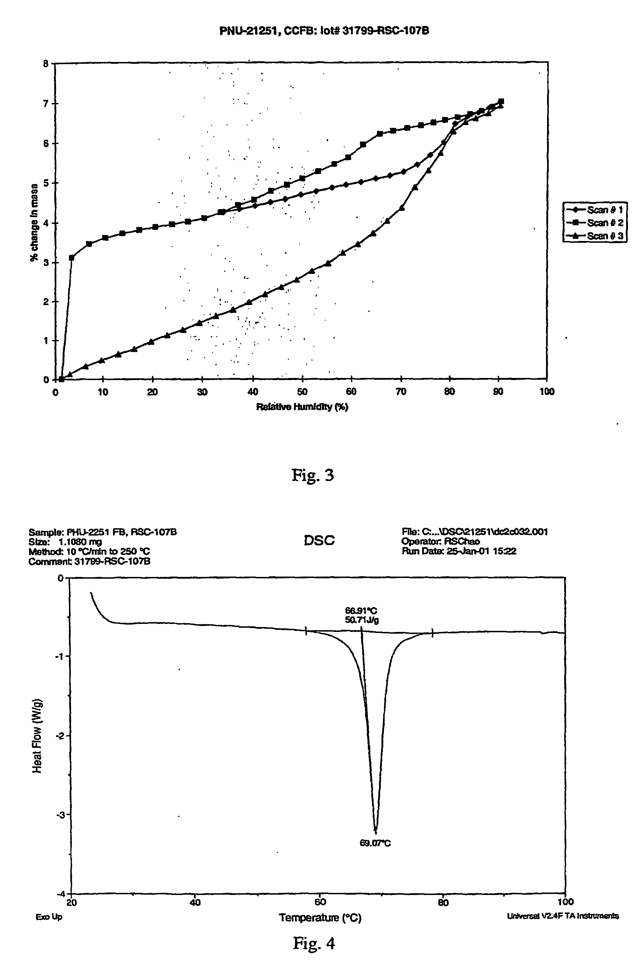

Figure 3 is the DMSG result of the isothermal (at 25°C) DMSG study of the crystalline clindamycin free base Form-I material. At the start of the run, the Form I material consists of approximately 4% water, which is consistent to a monohydrate structure. In the first scan, it retains the moisture content water content from ambient up to 70%RH, from 70%RH to 90%RH it sorbed about 2% moisture. Since PXRD of the wet material yields the same pattern as the starting Form I material, the sorbed moisture is likely on the surface, and not involved in the crystal lattice. The second scan indicates that the slow release of moisture from the material as the humidity drops from 90%RH down to 6%RH under the drying condition tested (90 -> 6% RH at 25 C). Below 6% RH, the material loses its hydrate water content and becomes amorphous material, verified by the PXRD. The third scan indicates that the amorphous clindamycin free base remains its non-crystalline nature from 0%RH to 80%RH; above 80%RH the material becomes crystalline clindamycin free base.

From the 3-scan DMSG result, it is evident that crystalline clindamycin free base Form I is stable at moderate humidity condition (10% - 90% RH); however it could lose the hydrate water and converts to the amorphous or non-crystalline CFB at the extremely low relative humidity ranges (below 6%RH).

### EXAMPLE 4

This example illustrates the characterization of the thermal behavior of crystalline clindamycin free base Form I using differential scanning calorimetry (DSC).

DSC study was performed using a TA Instruments model 2920 module with a Thermal Analyst 5000 controller (TA Instruments, Wilmington DE). Data were collected and analyzed using TA Instruments Thermal Solutions for NT and Universal Analysis for NT software.

A sample of about 1-mg was accurately weighed into an aluminum pan with lid, which was crimped to ensure good thermal contact. Unless specified otherwise, the samples were heated using a linear ramp rate of 10 °C/min from ambient to approximately 300°C. The DSC cell was purged at 50 ± 5 cc/min dry nitrogen (AGA Model FM 1050 ball float meter/controller). The DSC cell constant was determined from the enthalpy of fusion of pure indium and the temperature calibration was a single point correction based on the melting point of indium. Figure 4 displays a thermogram for crystalline clindamycin free base Form-I material, exhibiting a strong melting endotherm at 69.1°C (peak temperature) with a heat of fusion of 50.7 J/g. No other transition was evident. The closeness between the onset temperature of 66.9°C and peak temperature of 69.1°C for the endotherm suggests the material is predominately crystalline. No amorphous clindamycin was detected in the sample by this technique.

### EXAMPLE 5

This example illustrates a method of preparation of crystalline clindamycin free base, Form II upon addition of clindamycin hydrochloride solution to an aqueous solution of NaOH and ethanol.

Crystalline clindamycin free base, Form II, was generated by pilot plant run (10 kg scale) as follows:

A clindamycin hydrochloride solution was prepared by dissolving 10kg of Clindamycin HCl in 30kg water (3:1 ratio). This served as the substrate solution, prepared in the receiver.

In the crystallizer, 36.8kg water, 1.83 kg 50% aqueous sodium hydroxide (10% excess) and 6 kg absolute ethyl alcohol (10% of final slurry volume) were combined.

Using a Masterflex pump head and tubing, the clindamycin HCl substrate solution was delivered from the receiver into the solution in the crystallizer while maintaining the crystallizer pot temperature at 20° to 24° C with very high agitation to help prevent agglomeration of chunks.

The substrate delivery schedule was obtained by appropriate pump settings according to the following schedule: (a) for the first hour, approximately 750g (2%) were added to form seed crystals; (b) for the 2^{nd} hour, approximately 3.8kg (10%) were added to form abundant seed base and (c) for the final 1hr, the remainder of the substrate (approximately 30.4kg, or 88%) was added.

The resulting slurry was stirred for 1 hour at 20° to 24°C and then filter. The cake was washed with 50 to 100 kg of water at same temperature range until pH of filtrate is less than 9. The free liquid was then blown off for 5 minutes and the cake was dried with humidified drying using an apovac with water as seal fluid.

Humidity was maintained by using 40°C nitrogen and 20° condenser set point. The material was dried to constant weight. Under these conditions, the final L.O.D. was approximately 4.5%, which would be expected for the monohydrate material. After drying, the material was processed through a comil and packaged.

The crystallinity and crystal form were verified by powder X-ray diffraction.

### EXAMPLE 6

This example illustrates the powder x-ray diffraction analysis of crystalline clindamycin free base, Form II.

Figure 5 shows the X-ray diffraction pattern of Form II crystalline clindamycin freebase prepared as in example 5. The crystalline clindamycin free base, Form II, produced strong crystalline diffraction signals. The appearance of multiple strong and sharp diffraction peaks of Figure 5 indicates good crystallinity of the Form II material.

There is no evidence that any amorphous material is present in the sample such that if any is present it is no more than a minor component.

### EXAMPLE 7

This example illustrates the moisture sorption gravimetry (DMSG) of crystalline clindamycin free base, Form II.

DMSG analysis of crystalline clindamycin free base, Form-II was performed using an isothermally controlled atmospheric microbalance to study the moisture uptake of crystalline clindamycin free base Form-II and its stability at various humidities. Approximately 10 mg samples were used in the microbalance, samples were run as received. The humidity was set at ambient conditions on the day analysis began. The DMSG analysis of crystalline clindamycin free base Form II consisted of four scans: ambient to 0% RH, 0% RH to 90% RH, 90% RH to 0% RH, 0% RH to 90% RH. The scan rate for the DMSG analysis was 3 % RH/step. The mass was measured every two minutes and an equilibrium window of 5 points within 0.001 mg. The RH was changed to the next value (+/- 3%RH) when the mass of the sample was stable to within 0.001 mg.

Figure 6 displays the DMSG result of the crystalline clindamycin free base Form-II material. At the start of the run, the Form II material consists of approximately 2% water, which is consistent to a hemi-hydrate structure. In the first scan, it retains the moisture content as the humidity decreased from ambient to 10%RH, below 10% RH it quickly loses all moisture content and becomes amorphous. The second scan indicates that the amorphous clindamycin free base remains its amorphous nature from 0%RH up to 80%RH; at 80%RH the material has sorbed about 2% of moisture and slowly becomes crystalline clindamycin free base as it reaches 90%RH. The sorption content suggests that the crystals lattice allows only about 2% moisture, to fill the hemi-hydrate structural space. The third scan illustrates the material retains the moisture content as the humidity drops from 90%RH down to 10%RH; as RH dropped below 6%RH the material loses all water content and becomes amorphous material again. The fourth scan, from 0% up to 60%RH, reproduces similar sorption profile as the second scan, which indicates that the amorphous clindamycin free base remains its non-crystalline nature from 0%RH to 60%RH.

From the 4-scan DMSG-F2 result, it is evident that crystalline clindamycin free base Form II retains about 2% water content at moderate humidity condition (10% - 80% RH); however it could lose the hydrate water and converts to the amorphous or non-crystalline CFB at the extremely low relative humidity ranges (below 6%RH).

### EXAMPLE 8

This example illustrates the characterization of the thermal behavior of crystalline clindamycin free base Form II using differential scanning calorimetry (DSC).

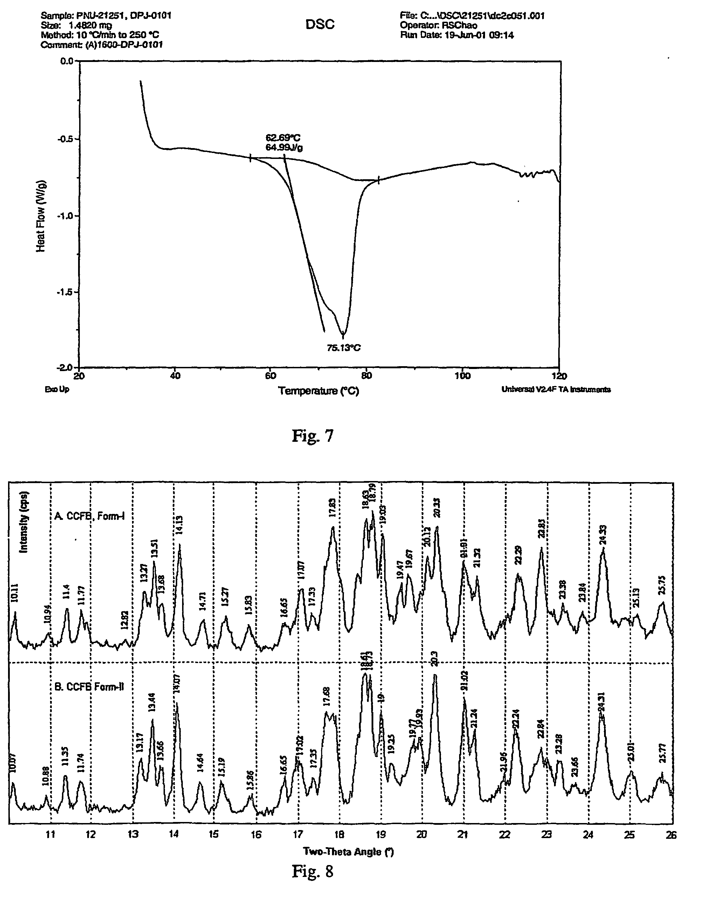

Differential scanning calorimetry was performed as indicated in Example 4. Figure 7 displays a representative thermogram for crystalline clindamycin free base, Form II material. The plot reveals a strong and broad melting endotherm with extrapolated onset at 62.7°C and peak temperature at 75.1°C with a heat of fusion of 65.0 J/g. No other transition was evident.

An unusually large separation occurs between the onset (62.7°C) and peak (75.1°C) temperatures of the broad endotherm as temperature approaches complete melting temperature. The Form II material has been processed with 10% ethanol as co-solvent and assay for residual solvent assay reported that the representative Form II sample yields 1.03% ethanol. Therefore, the broad endotherm may be due to release of both water and ethanol from the material as the complete melting temperature is approached.

Whether amorphous clindamycin present in the material was not detectable by this technique.

### EXAMPLE 9

The powder X-ray diffraction patterns for Forms I and II were found to be somewhat similar, as illustrated in Figure 8. Careful examination, however, reveals that the two forms differ, particularly in the spectral range of 18-24 two-theta angles as shown in the figure. Note that peaks 19.47, 20.12, and 22.85 in the Form I spectrum are either not present or of low intensity in the Form II spectrum.

In the DSC studies, Forms I and II were shown to differ in their distinct peak temperature differences: Form-I at 69°C, while Form-II at 75°C.

In the DMSG studies, the moisture sorption and residual solvents tests reveals that the Form I contains about 4% of water, which is consistent with the material being a monohydrate, while Form II contains less water, but with small ethanol content (1.0-1.4%). The subtle differences between crystalline clindamycin free base Forms I and II can be reasoned as the small ethanol content in the Form II lattice and causes minor structural changes thus its powder PXRD features. Further studies have shown the Form II will convert to Form I upon trituration in aqueous media, or at stressed humidity conditions

These data, taken together, indicate that Forms I and II are of the same polymorphic form, but differ due to the presence on a small amount of ethanol in the Form II material. The Form II material is, therefore, considered to be a solvate form or pseudopolymorphic form.

### EXAMPLE 10

This example illustrates a method of preparation of crystalline clindamycin free base, Form III upon addition of clindamycin hydrochloride solution to an aqueous solution of NaOH and ethanol.

A sample of crystalline clindamycin free base, Form III, was generated by lab run (40g scale) as follows:

A clindamycin hydrochloride solution was prepared by dissolving 40g clindamycin HCl in 120 ml of water (3:1)

In the crystallizer, 34.8 ml of a 10% sodium hydroxide aqueous solution (10% excess), 144 ml water with no co-solvent and 1.99g seed crystals (5% seeding), produced as described in Example 5, above, were combined.

The Clindamycin HCl substrate solution was delivered into the crystallizer *via* syringe pump and tubing using the following feed schedule: (a) 4.4 ml per hour were delivered until 30 ml (20%) has been added and thereafter (b) 8.8 ml per hour were delivered. The total combined feed time of both feed stages was approximately 20 hours.

The pot temp was maintained at room temperature (21° to 23.5° C) throughout.

Agitation was moderately high at 300rpm in a 500ml 3NRB Flask.

After the addition to the crystallizer is complete, the mixture was stirred for 1hr. 45min. at room temp and filtered.

The filtered material was then dried overnight by pulling filtered ambient air through the cake using full house vacuum, which approximates humidified drying.

The crystallinity of the resulting sample of crystalline clindamycin free base Form III was verified by powder X-ray diffraction.

### EXAMPLE 11

This example illustrates the powder X-ray diffraction analysis of crystalline clindamycin free base, Form III.

Figure 9 shows the powder X-ray of crystalline clindamycin free base, Form III prepared as described in Example 9. As is seen in the figure, Form III, produced strong crystalline diffraction signals. The appearance of multiple strong and sharp diffraction peaks of Figure 9 indicates good crystallinity of the crystalline clindamycin free base, Form III.

There is no evidence that any amorphous material is present in the sample such that if any is present it is no more than a minor component.

With its unique powder X-ray diffraction pattern, Form III material can be readily differentiated from the other crystalline clindamycin free base solid forms, Forms I or II.

### EXAMPLE 12

This example illustrates the moisture sorption gravimetry (DMSG) of crystalline clindamycin free base, Form III.

DMSG analysis crystalline clindamycin free base, Form-III was performed using an isothermally controlled atmospheric microbalance to study the moisture uptake of crystalline clindamycin free base, Form-III and its stability at various humidities. Samples of approximately 10 mg were used in the microbalance. The humidity was set at ambient conditions on the day analysis began. The DMSG analysis of crystalline clindamycin free base Form III consisted of four scans: ambient to 0% RH, 0% RH to 90% RH, 90% RH to 0% RH, 0% RH to 90% RH. The scan rate for the DMSG analysis was 3 % RH/step. The mass was measured every two minutes and an equilibrium window of 5 points within 0.001 mg. The RH was changed to the next value (+/- 3%RH) when the mass of the sample was stable to within 0.001 mg.

Figure 10 demonstrates the DMSG result of the crystalline clindamycin free base Form-III material. At the start of the run, the Form III material consists of approximately 4.5% water, which is consistent with the material being a monohydrate. In the first scan, it slowly loses about 1.5% moisture content from ambient down to about 6%RH, from 6%RH down to 0% RH it quickly loses all moisture content and becomes amorphous. The second scan indicates that the amorphous clindamycin free base remains its amorphous nature from 0%RH to 80%RH; at 80%RH the material has sorbed about 6% of moisture and slowly becomes crystalline clindamycin free base. The sorption content suggests that the crystals at 90%RH has sorbed about 6% moisture. The third scan illustrates the material retained the moisture content as the humidity drops from 90%RH down to 6%RH; as RH dropped below 6%RH the material loses all water content and becomes amorphous material again. The fourth scan, from 0% up to 90%RH, reproduces similar sorption profile as the second scan, which indicates that the amorphous clindamycin free base remains in its non-crystalline nature from 0%RH to 80%RH, then slowly becomes crystalline clindamycin free base as the humidity approaches to 90%RH.

From the 4-scan DMSG-F3 result, it is evident that crystalline clindamycin free base Form III retains about 4.5% water content (+/- 1%) at moderate humidity condition (10% - 80% RH). Crystalline clindamycin free base Form III material could lose all its water content and becomes amorphous or non-crystalline clindamycin free base at the extremely low relative humidity ranges (below 6%RH).

### EXAMPLE 13

This example illustrates the characterization of the thermal behavior of crystalline clindamycin free base Form III using differential scanning calorimetry (DSC).

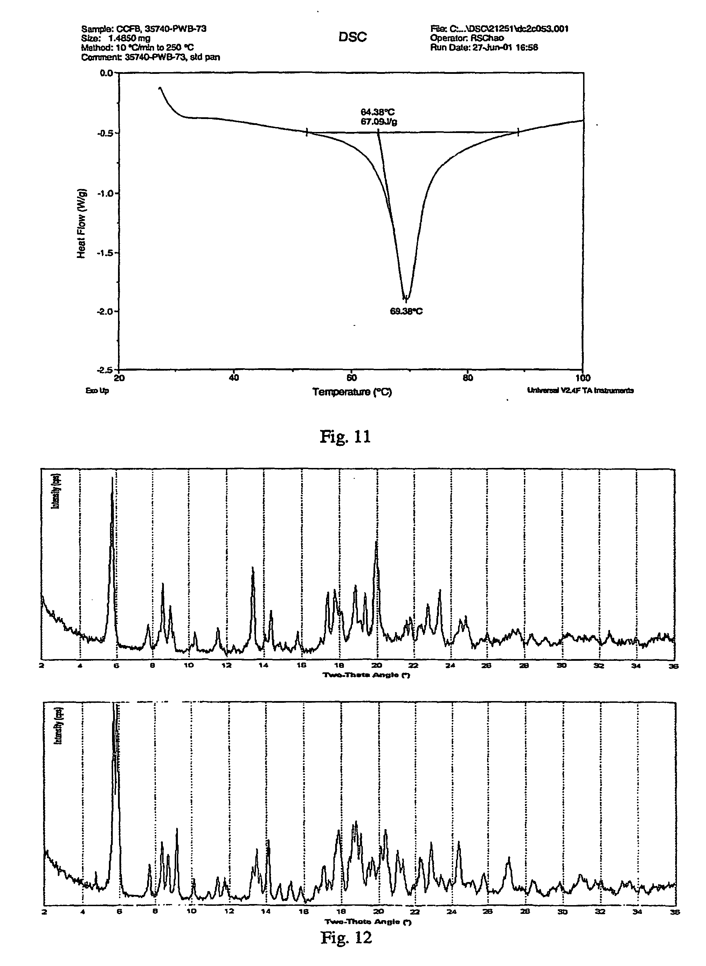

Differential scanning calorimetry was performed as described in Example 4. Figure 11 shows a representative thermogram for crystalline clindamycin free base Form-III material. The plot reveals a strong melting endotherm having an extrapolated onset temperature of 64.4°C and a peak temperature of 69.4°C with a heat of fusion of 67.1J/g. No other transition was evident. The moderate separation between the onset (64.4°C) and peak (69.4°C) temperatures of the endotherm suggests the material may release water slower than the Form I material prior to the complete melt.

Whether amorphous clindamycin present in the material was not detectable by this technique.

### EXAMPLE 14

Comparison of the X-ray diffraction patterns of Forms I and III as shown in Figure 12 reveals that the spectra are substantially different. For example, the Form I crystalline material shows peaks at two-theta angles of about 9.3, 11.7, 14.1,19.0, 20.4, 21.0, 22.3, 24.4, 25.8, and 27.1 which are not present or of low intensity in the Form III spectrum whereas the Form III crystalline material shows peaks at two-theta angles of about 14.4, 17.4, 19.4, 20.0 and 23.4 which are not present or of low intensity in the Form I spectrum. It is, therefore, concluded that Forms I and III are different polymorphic forms of crystalline clindamycin free base.

### EXAMPLE 15

This example illustrates the different powder X-ray diffraction patterns of clindamycin free base as amorphous material, crystalline Type I material and a 50% mixture of amorphous and crystalline Form I.

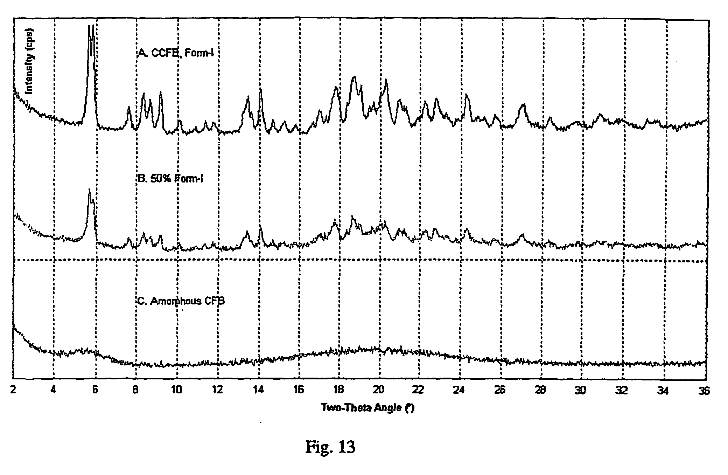

Amorphous and crystalline Form I clindamycin free base were obtained as described in Example 1. These materials were considered to be phase pure, i.e. approximately 100% amorphous or approximately 100% Form I crystalline material. A mixture was formed by combining the two in equal portions to obtain a material which contained 50% Form crystalline material. Powder X-ray diffraction patterns were then obtained for each of the 100% amorphous, 100% Form I, crystalline and 50% mixture samples. The three spectra are shown in Figure 13 plotted on the same intensity scale. As shown in the figure the 50% mixture gave similar peaks as were observed from the Type I, but at a lower intensity.

### EXAMPLE 16

This example illustrates the use of methanol co-solvent in the crystallization of clindamycin free base.

In this approach clindamycin free base is crystallized upon adding clindamycin hydrochloride solution by slow infusion to an alkaline/co-solvent solution containing NaOH and methanol as co-solvent.

An aqueous alkaline/co-solvent solution in which the co-solvent was 20% methanol was prepared by combining 8.7 ml of 10% aqueous NaOH, 15 ml methanol and 21 ml water in a 250 ml vessel in which the solution was stirred at 400 rpm and maintained at 21°-23°C. A solution of clindamycin hydrochloride was prepared by dissolving 10.04 g in 30 ml water. The clindamycin hydrochloride solution was then loaded into a syringe pump and infused into the NaOH/methanol solution over a period 91 minutes. At approximately 30 minutes, a hazy liquid phase began to form along with some oil that quickly coalesced and settled. At 33 minutes, the oil suddenly crystallized with a slight exotherm. The mixture became a suspension of fine white particles and small white chunks.

The mixture was stirred for an additional 82 minutes after the end of infusion and then filtered under reduced pressure in a Buchner funnel and washed with 50 ml water. The wet weight was 9.832 g with a cake depth of about 12 mm and volume of about 17 ml. The material was then dried in a oven at 40°C for about 60 hours. The yield was 8.13 grams or 91.6% of theoretical yield.

### EXAMPLE 17

This example illustrates the use NaOAc·3H₂O instead of an alcohol co-solvent in the crystallization of clindamycin free base.

A solution was prepared by combining 34.8 ml of 10% aqueous NaOH, 7.07 g NaOAc·3H₂O and 144 ml water. The solution was stirred at 400 rpm and maintained at 22±2°C. A solution of clindamycin hydrochloride was prepared by dissolving 40 g in 120 ml water. The clindamycin hydrochloride solution was added to the aqueous alkaline solution in an infusion schedule of 3.3 ml/hr for 3.0 hr, 11.2 ml/hr for 1.15 hr and 0.68 ml/min for 0.25 hr.

The precipitate was filtered in a Buchner funnel under reduced pressure, washed 4 times with 50 ml water and dried in ambient air. Yield was 30.98 g.

### EXAMPLE 18

This example illustrates the use of sodium bicarbonate instead of NaOH in crystallization of clindamycin free base from the hydrochloride salt.

An aqueous alkaline/co-solvent solution was prepared by combining 1.93 g NaHCO₃, with 7.5 ml absolute ethanol and 37.2 ml water. The solution was stirred at 400 rpm and maintained at 22±2°C. A solution of clindamycin hydrochloride was prepared by dissolving 10.04 g in 30 ml water and adding to the ethanol/NaHCO₃ solution by an infusion schedule of 0.71 ml/hr for 65, 3.5 ml/hr for about 35 min and 0.48 ml/hr for about 40 min at which time all of solution had been infused. Fine solids began to appear after one hour and a thin slurry of fine solids formed at one hour and 30 minutes. The precipitate was filtered under reduced pressure in a Buchner funnel and washed with 50 ml water. Reduced pressure was maintained for an additional 6 min. The precipitate was dried in a vacuum oven at 40°C. Yield was 7.017 or 79.16 of theoretical yield.

A second crop was obtained by adding an add ional 1.58 g NaHCO₃ and stirring under vacuum. The precipitate was filtered, washed and dried in vacuum oven to give an additional yield of 1.2127 or 13.68% of the theoretical yield. A third crop of 0.1177g or 1.33% of theoretical yield was obtained upon addition of 8.7 ml of 10% NaOH.

All references cited in this specification are hereby incorporated by reference. The discussion of the references herein is intended merely to summarize the assertions made by their authors and no admission is made that any reference constitutes prior art relevant to patentability. Applicants reserve the right to challenge the accuracy and pertinency of the cited references.

### EXAMPLE 19

This example illustrates a method of preparation of crystalline clindamycin free base, Form I upon addition of clindamycin hydrochloride solution to an aqueous solution of NaOH and ethanol.

Crystalline clindamycin free base, Form II, was generated by pilot plant run (10 kg scale) as follows:

A clindamycin hydrochloride solution was prepared by dissolving 10kg of Clindamycin HCl in 30kg water (3:1 ratio). This served as the substrate solution, prepared in the receiver.

In the crystallizer, 36.8kg water, 1.83 kg 50% aqueous sodium hydroxide (10% excess) and 6 kg absolute ethyl alcohol (10% of final slurry volume) were combined.

Using a Masterflex pump head and tubing, the clindamycin HCl substrate solution was delivered from the receiver into the solution in the crystallizer while maintaining the crystallizer pot temperature at 20° to 24° C with very high agitation to help prevent agglomeration of chunks.

The substrate delivery schedule was obtained by appropriate pump settings according to the following schedule: (a) for the first hour, approximately 750g (2%) were added to form seed crystals; (b) over the next 2 hours, approximately 3.8kg (10%) were added to form abundant seed base and (c) over the final 1.5hr, the remainder of the substrate (approximately 30.4kg, or 88%) was added.

The resulting slurry was stirred for 1 hour at 20° to 24°C and then filter. The cake was washed with 50 to 100 kg of water at same temperature range until pH of filtrate is less than 9. The cake was then held in contact with five successive 15 kg portions of water for a minimum of four hours with each portion. The free liquid was then blown off for 5 minutes and the cake was dried with humidified drying using an apovac with water as seal fluid.

Humidity was maintained by using 40°C nitrogen and 20° condenser set point. The material was dried to constant weight. Under these conditions, the final L.O.D. was approximately 4.5%, which would be expected for the monohydrate material. After drying, the material was processed through a comil and packaged.

The crystallinity and crystal form were verified by powder X-ray diffraction.

## Claims

1. A substantially crystalline clindamycin free base.

2. The substantially crystalline clindamycin free base of claim 1, **characterized by** bright birefringence using polarized microscopic inspection or by sharp peaks using powder x-ray diffraction.

3. The substantially crystalline clindamycin free base of claim 1 which is substantially all Form I.

4. The substantially crystalline clindamycin free base of claim 3 which is **characterized by** an X-ray powder diffraction pattern substantially as shown in Figure 1.

5. The substantially crystalline clindamycin free base of claim 3 which is **characterized by** a differential scanning calorimetry exhibiting an endotherm having an extrapolated onset temperature of about 67°C, a peak temperature of about 69°C and an associated heat of about 51 J/g.

6. The substantially crystalline clindamycin free base of claim 1 which is substantially all Form II.

7. The substantially crystalline clindamycin free base of claim 6 which is **characterized by** an X-ray powder diffraction pattern substantially as shown in Figure 5.

8. The substantially crystalline clindamycin free base of claim 6 which is **characterized by** a differential scanning calorimetry exhibiting an endotherm having an extrapolated onset temperature of about 62.7°C, a peak temperature of about 75.1°C and an associated heat of about 65.0 J/g.

9. The substantially crystalline clindamycin free base of claim 1 which is substantially all Form III.

10. The substantially crystalline clindamycin free base of claim 9 which is **characterized by** an X-ray powder diffraction pattern substantially as shown in Figure 9.

11. The substantially crystalline clindamycin free base of claim 9 which is **characterized by** a differential scanning calorimetry exhibiting an endotherm having an extrapolated onset temperature of about 64.4°C, a peak temperature of about 69.4°C and an associated heat of about 69.4 J/g.

12. A clindamycin free base drug substance, comprising about 10% (w/w) to about 100% (w/w) of the substantially crystalline clindamycin free base of any of claims 1-11.

13. The drug substance of claim 12, comprising about 60% (w/w) to about 100% (w/w) of the substantially crystalline clindamycin free base.

14. A pharmaceutical composition comprising at least about 1% (w/w) of the substantially crystalline clindamycin free base of any of claims 1-11 in a pharmaceutically acceptable formulation.

15. The pharmaceutical composition of claim 14, in an extended release dosage form.

16. The use of a substantially crystalline clindamycin free base for the manufacture of a pharmaceutical composition of claim 14 to treat a bacterial infection in a subject.

17. A process for preparing a crystalline clindamycin free base, comprising the steps of:
(a) providing an aqueous solution containing a clindamycin salt;
(b) adding an alkali to the solution to form a precipitate comprising an amorphous free base; and
(c) crystallizing crystalline clindamycin free base from the precipitate.

18. The process of claim 17, wherein the clindamycin salt provided in step (a) is clindamycin hydrochloride.

19. The process of claim 17, wherein the alkali added in step (b) is selected from the group consisting of NaOH and NaHCO₃.

20. The process of claim 17, wherein the clindamycin free base is crystallized from the precipitate in step (c) by agitating, sonicating, or by both agitating and sonicating the precipitate in the solution.

21. The process of any of claims 17-20, wherein substantially all of the crystalline clindamycin free base is Form I.

22. The process of any of claims 17-20, wherein substantially all of the crystalline crystalline clindamycin free base is Form II.

23. The process of any of claims 17-20, wherein at least about 10% (w/w) of the crystalline clindamycin free base is Form III.

24. A process for preparing crystalline clindamycin free base, comprising the steps of:
(a) providing an aqueous alkali solution containing a water-soluble organic substance and an alkali;
(b) adding to the aqueous alkali solution an aqueous solution of a clindamycin salt, and
(c) allowing crystals of crystalline clindamycin free base to form in the solution.

25. The process of claim 24 wherein the water-soluble organic substance comprises an alcohol.

26. The process of claim 25 wherein the alcohol is methanol or ethanol.

27. The process of any of claims 24-26, wherein the clindamycin salt added in step (b) is clindamycin hydrochloride.

28. The process of any of claims 24-26, wherein the aqueous alkali solution comprises NaOH or NaHCO₃.

29. The process of claim 24, further comprising a step of isolating the crystalline free base.

30. The process of claim 29, wherein at least substantially all of the isolated crystalline clindamycin free base is Form I.

31. The process of claim 30 wherein the isolated crystalline clindamycin free base is **characterized by** an X-ray powder diffraction pattern substantially as shown in Figure 1.

32. The process of claim 30 wherein the isolated crystalline clindamycin free base is **characterized by** a differential scanning calorimetry exhibiting an endotherm having an extrapolated onset temperature of about 66.9°C, a peak temperature of about 69.1°C and an associated heat of about 50.7 J/g.

33. The process of claim 29, wherein substantially all of the isolated crystalline clindamycin free base is Form II.

34. The process of claim 33 wherein the isolated crystalline clindamycin free base is **characterized by** an X-ray powder diffraction pattern substantially as shown in Figure 5.

35. The process of claim 34 wherein the isolated crystalline clindamycin free base is **characterized by** a differential scanning calorimetry exhibiting an endotherm having an extrapolated onset temperature of about 62.7°C, a peak temperature of about 75.1°C and an associated heat of about 65.0 J/g.

36. The process of claim 29, wherein at least about 10% (w/w) of the isolated crystalline clindamycin free base is Form III.

37. The process of claim 36 wherein the isolated crystalline clindamycin free base is **characterized by** an X-ray powder diffraction pattern substantially as shown in Figure 9.

38. The process of claim 36 wherein the isolated crystalline clindamycin free base is **characterized by** a differential scanning calorimetry exhibiting an endotherm having an extrapolated onset temperature of about 64.4°C, a peak temperature of about 69.4°C and an associated heat of about 69.4 J/g.

## Patentansprüche

1. Im Wesentlichen kristalline freie Base von Clindamycin.

2. Im Wesentlichen kristalline freie Base von Clindamycin nach Anspruch 1, **gekennzeichnet durch** helle Doppelbrechung bei polarisierter mikroskopischer Untersuchung oder **durch** scharfe Peaks bei Röntgen-Pulverdiffraktion.

3. Im Wesentlichen kristalline freie Base von Clindamycin nach Anspruch 1, welche im Wesentlichen vollständig in der Form I vorliegt.

4. Im Wesentlichen kristalline freie Base von Clindamycin nach Anspruch 3, charakterisiert durch ein Röntgen-Pulverdiffraktionsmuster im Wesentlichen wie in Figur 1 dargestellt.

5. Im Wesentlichen kristalline freie Base von Clindamycin nach Anspruch 3, charakterisiert durch eine Differentialscanning-Kalorimetrie, die Endothermie zeigt mit einer extrapolierten Onsettemperatur von etwa 67°C, einer Peaktemperatur von etwa 69°C und einer assoziierten Wärme von etwa 51 J/g.

6. Im Wesentlichen freie Base von Clindamycin nach Anspruch 1, welche im Wesentlichen vollständig in der Form II vorliegt.

7. Im Wesentlichen kristalline freie Base von Clindamycin nach Anspruch 6, charakterisiert durch ein Röntgen-Pulverdiffraktionsmuster im Wesentlichen wie in Figur 5 dargestellt.

8. Im Wesentlichen kristalline freie Base von Clindamycin nach Anspruch 6, charakterisiert durch eine Differentialscanning-Kalorimetrie, die Endothermie zeigt mit einer extrapolierten Onsettemperatur von etwa 62,7°C, einer Peaktemperatur von etwa 75,1°C und einer assoziierten Wärme von etwa 65,0 J/g.

9. Im Wesentlichen freie Base von Clindamycin nach Anspruch 1, welche im Wesentlichen vollständig in der Form III vorliegt.

10. Im Wesentlichen kristalline freie Base von Clindamycin nach Anspruch 9, charakterisiert durch ein Röntgen-Pulverdiffraktionsmuster im Wesentlichen wie in Figur 9 dargestellt.

11. Im Wesentlichen kristalline freie Base von Clindamycin nach Anspruch 9, charakterisiert durch eine Differentialscanning-Kalorimetrie, die Endothermie zeigt mit einer extrapolierten Onsettemperatur von etwa 64,4°C, einer Peaktemperatur von etwa 69,4°C und einer assoziierten Wärme von etwa 69,4 J/g.

12. Eine freie Base von Clindamycin darstellender Arzneimittelwirkstoff, umfassend etwa 10 Gew.% bis etwa 100 Gew.% der im Wesentlichen kristallinen freien Base von Clindamycin nach einem der Ansprüche 1 - 11.

13. Arzneimittelwirkstoff nach Anspruch 12, umfassend etwa 60 Gew.% bis etwa 100 Gew.% der im Wesentlichen kristallinen freien Base von Clindamycin.

14. Pharmazeutische Komposition, umfassend wengistens etwa 1 Gew.% der im Wesentlichen kristallinen freien Base von Clindamycin nach einem der Ansprüche 1 - 11 in einer pharmazeutisch akzeptablen Formulierung.

15. Pharmazeutische Komposition nach Anspruch 14 in einer Präparatform mit verzögerter Wirkstoffabgabe.

16. Verwendung einer im Wesentlichen kristallinen freien Base von Clindamycin zur Herstellung der pharmazeutischen Komposition nach Anspruch 14 zur Behandlung einer bakteriellen Infektion bei einem Patienten.

17. Verfahren zur Herstellung einer kristallinen freien Base von Clindamycin, umfassend die Schritte:
a) Bereitstellung einer wässrigen Lösung eines Clindamycinsalzes;
b) Zugabe von Alkali zur Lösung unter Bildung eines Niederschlags, der die amorphe freie Base enthält; und
c) Kristallisation der kristallinen freien Base des Clindamycin aus dem Niederschlag.

18. Verfahren nach Anspruch 17, wobei das Clindamycinsalz in Schritt a) Clindamycinhydrochlorid darstellt.

19. Verfahren nach Anspruch 17, wobei das in Schritt b) zugegebene Alkali ausgewählt ist aus NaOH und NaHCO₃.

20. Verfahren nach Anspruch 17, wobei die freie Base von Clindamycin in Schritt c) aus dem Niederschlag kristallisiert wird durch Rühren oder Ultraschallbehandlung oder durch sowohl Rühren als auch Ultraschallbehandlung des Niederschlags in der Lösung.

21. Verfahren nach einem der Ansprüche 17 - 20, wobei im Wesentlichen die gesamte kristalline freie Base von Clindamycin in der Form I erhalten wird.

22. Verfahren nach einem der Ansprüche 17 - 20, wobei im Wesentlichen die gesamte kristalline freie Base von Clindamycin in der Form II erhalten wird.

23. Verfahren nach einem der Ansprüche 17 - 20, wobei wenigstens etwa 10 Gew.% der kristallinen freien Base von Clindamycin in der Form III erhalten werden.

24. Verfahren zur Herstellung einer kristallinen freien Base von Clindamycin, umfassend die Schritte:
a) Bereitstellen einer wässrigen Alkalilösung, enthaltend eine wasserlösliche organische Substanz und Alkali;
b) Zugabe einer wässrigen Lösung eines Clindamycinsalzes zur wässrigen Alkalilösung;
c) Bildung von Kristallen der kristallinen freien Base von Clindamycin in der Lösung.

25. Verfahren nach Anspruch 24, wobei die wasserlösliche organische Substanz einen Alkohol umfasst.

26. Verfahren nach Anspruch 25, wobei der Alkohol Methanol oder Ethanol darstellt.

27. Verfahren nach einem der Ansprüche 24 - 26, wobei das in Schritt b) zugesetzte Clindamycinsalz Clindamycinhydrochlorid ist.

28. Verfahren nach einem der Ansprüche 24 - 26, wobei die wässrige Alkalilösung NaOH oder NaHCO₃ enthält.

29. Verfahren nach Anspruch 24, weiterhin umfassend die Isolierung der kristallinen freien Base.

30. Verfahren nach Anspruch 29, wobei wenigstens im Wesentlichen die gesamte isolierte kristalline freie Base von Clindamycin in der Form I ist.

31. Verfahren nach Anspruch 30, wobei die isolierte kristalline freie Base von Clindamycin charakterisiert ist durch ein Röntgen-Pulverdiffraktionsmuster im Wesentlichen wie in Figur 1 dargestellt.

32. Verfahren nach Anspruch 30, wobei die isolierte kristalline freie Base von Clindamycin charakterisiert ist durch eine Differentialscanning-Kalorimetrie, die Endothermie zeigt mit einer extrapolierten Onsettemperatur von etwa 66,9°C, einer Peaktemperatur von etwa 69,1°C und einer assoziierten Wärme von etwa 50,7 J/g.

33. Verfahren nach Anspruch 29, wobei im Wesentlichen die gesamte isolierte kristalline freie Base von Clindamycin in der Form II ist.

34. Verfahren nach Anspruch 33, wobei die isolierte kristalline freie Base von Clindamycin charakterisiert ist durch ein Röntgen-Pulverdiffraktionsmuster im Wesentlichen wie in Figur 5 dargestellt.

35. Verfahren nach Anspruch 34, wobei die isolierte kristalline freie Base von Clindamycin charakterisiert ist durch eine Differentialscanning-Kalorimetrie, die Endothermie zeigt mit einer extrapolierten Onsettemperatur von etwa 62,7°C, einer Peaktemperatur von etwa 75,1 °C und einer assoziierten Wärme von etwa 65,0 J/g.

36. Verfahren nach Anspruch 29, wobei wenigstens etwa 10 Gew.% der isolierten freien Base von Clindamycin in der Form III sind.

37. Verfahren nach Anspruch 36, wobei die isolierte kristalline freie Base von Clindamycin charakterisiert ist durch ein Röntgen-Pulverdiffraktionsmuster im Wesentlichen wie in Figur 9 dargestellt.

38. Verfahren nach Anspruch 36, wobei die isolierte kristalline freie Base von Clindamycin charakterisiert ist durch eine Differentialscanning-Kalorimetrie, die Endothermie zeigt mit einer extrapolierten Onsettemperatur von etwa 64,4°C, einer Peaktemperatur von etwa 69,4°C und einer assoziierten Wärme von etwa 69,4 J/g.

## Revendications

1. Clindamycine sensiblement cristalline, à l'état de base libre.

2. Clindamycine sensiblement cristalline, à l'état de base libre, selon la revendication 1, **caractérisée par** une biréfringence brillante en utilisant une inspection microscopique polarisée ou par des pics aigus en utilisant une diffraction des rayons X sur poudre.

3. Clindamycine sensiblement cristalline, à l'état de base libre, selon la revendication 1, qui est sensiblement toute de Forme I.

4. Clindamycine sensiblement cristalline, à l'état de base libre, selon la revendication 3, qui est **caractérisée par** un tracé de diffraction des rayons X sur poudre sensiblement tel que représenté à la Figure 1.

5. Clindamycine sensiblement cristalline, à l'état de base libre, selon la revendication 3, qui est **caractérisée par** une calorimétrie différentielle à balayage manifestant un endotherme ayant une température de début extrapolée d'environ 67°C, une température de pic d'environ 69°C et une chaleur associée d'environ 51 J/g.

6. Clindamycine sensiblement cristalline, à l'état de base libre, selon la revendication 1, qui est sensiblement toute de Forme II.

7. Clindamycine sensiblement cristalline, à l'état de base libre, selon la revendication 6, qui est **caractérisée par** un tracé de diffraction des rayons X sur poudre sensiblement tel que représenté à la Figure 5.

8. Clindamycine sensiblement cristalline, à l'état de base libre, selon la revendication 6, qui est **caractérisée par** une calorimétrie différentielle à balayage manifestant un endotherme ayant une température de début extrapolée d'environ 62,7°C, une température de pic d'environ 75,1°C et une chaleur associée d'environ 65,0 J/g.

9. Clindamycine sensiblement cristalline, à l'état de base libre, selon la revendication 1, qui est sensiblement toute de Forme III.

10. Clindamycine sensiblement cristalline, à l'état de base libre, selon la revendication 9, qui est **caractérisée par** un tracé de diffraction des rayons X sur poudre sensiblement tel que représenté à la figure 9.

11. Clindamycine sensiblement cristalline, à l'état de base libre, selon la revendication 9, qui est **caractérisée par** une calorimétrie différentielle à balayage manifestant un endotherme ayant une température de début extrapolée d'environ 64,4°C, une température de pic d'environ 69,4°C et une chaleur associée d'environ 69,4 J/g.

12. Clindamycine, à l'état de base libre, comme substance médicamenteuse, comprenant d'environ 10% (poids/poids) à environ 100% (poids/poids) de la clindamycine sensiblement cristalline, à l'état de base libre, selon l'une quelconque des revendications 1 à 11.

13. Substance médicamenteuse selon la revendication 12, comprenant d'environ 60% (poids/poids) à environ 100% (poids/poids) de la clindamycine sensiblement cristalline, à l'état de base libre.

14. Composition pharmaceutique comprenant au moins environ 1% (poids/poids) de la clindamycine sensiblement cristalline, à l'état de base libre, selon l'une quelconque des revendications 1 à 11 dans une formulation pharmaceutiquement acceptable.

15. Composition pharmaceutique selon la revendication 14, dans une forme dosée à libération prolongée.

16. Utilisation d'une clindamycine sensiblement cristalline, à l'état de base libre, pour la fabrication d'une composition pharmaceutique selon la revendication 14, pour traiter une infection bactérienne chez un sujet.

17. Procédé de préparation d'une clindamycine cristalline à l'état de base libre, comprenant les étapes suivantes :
(a) la fourniture d'une solution aqueuse contenant un sel de clindamycine ;
(b) l'addition d'une base à la solution pour former un précipité comprenant une base libre amorphe ; et
(c) la cristallisation de la clindamycine cristalline à l'état de base libre à partir du précipité.

18. Procédé selon la revendication 17, dans lequel le sel de clindamycine fourni à l'étape (a) est du chlorhydrate de clindamycine.

19. Procédé selon la revendication 17, dans lequel la base ajoutée à l'étape (b) est sélectionnée dans le groupe consistant en NaOH et NaHCO₃.

20. Procédé selon la revendication 17, dans lequel la clindamycine à l'état de base libre est cristallisée à partir du précipité à l'étape (c) par agitation, sonication ou à la fois agitation et sonication, du précipité dans la solution.

21. Procédé selon l'une quelconque des revendications 17-20, dans lequel sensiblement toute la clindamycine cristalline à l'état de base libre est de Forme I.

22. Procédé selon l'une quelconque des revendications 17-20, dans lequel sensiblement toute la clindamycine cristalline à l'état de base libre est de Forme II

23. Procédé selon l'une quelconque des revendications 17-20, dans lequel au moins environ 10% (poids/poids) de la clindamycine cristalline à l'état de base libre est de Forme III.

24. Procédé de préparation de clindamycine cristalline à l'état de base libre, comprenant les étapes suivantes :
(a) la fourniture d'une solution aqueuse d'une base contenant une substance organique hydrosoluble et une base ;
(b) l'adjonction à la solution aqueuse basique d'une solution aqueuse d'un sel de clindamycine ; et
(c) le fait de laisser les cristaux de clindamycine cristalline à l'état de base libre se former dans la solution.

25. Procédé selon la revendication 24, dans lequel la substance organique hydrosoluble comprend un alcool.

26. Procédé selon la revendication 25, dans lequel l'alcool est le méthanol ou l'éthanol.

27. Procédé selon l'une quelconque des revendications 24-26, dans lequel le sel de clindamycine ajouté à l'étape (b) est du chlorhydrate de clindamycine.

28. Procédé selon l'une quelconque des revendications 24-26, dans lequel la solution aqueuse basique comprend NaOH ou NaHCO₃.

29. Procédé selon la revendication 24, comprenant en outre une étape d'isolement de la base libre cristalline.

30. Procédé selon la revendication 29, dans lequel au moins sensiblement toute la clindamycine cristalline, à l'état de base libre, isolée, est de Forme I.

31. Procédé selon la revendication 30, dans lequel la clindamycine cristalline, à l'état de base libre, isolée, est **caractérisée par** un motif de diffraction des rayons X sur poudre sensiblement tel que représenté à la Figure 1.

32. Procédé selon la revendication 30, dans lequel la clindamycine cristalline, à l'état de base libre, isolée, est **caractérisée par** une calorimétrie différentielle à balayage manifestant un endotherme ayant une température de début extrapolée d'environ 66,9°C, une température de pic d'environ 69,1°C et une chaleur associée d'environ 50,7 J/g.

33. Procédé selon la revendication 29, dans lequel sensiblement toute la clindamycine cristalline, à l'état de base libre, isolée, est de Forme II.

34. Procédé selon la revendication 33, dans lequel la clindamycine cristalline, à l'état de base libre, isolée, est **caractérisée par** un motif de diffraction des rayons X sur poudre sensiblement tel que représenté à la Figure 5.

35. Procédé selon la revendication 34, dans lequel la clindamycine cristalline, à l'état de base libre, isolée, est **caractérisée par** une calorimétrie différentielle à balayage manifestant un endotherme ayant une température de début extrapolée d'environ 62,7°C, une température de pic d'environ 75,1°C et une chaleur associée d'environ 65,0 J/g.

36. Procédé selon la revendication 29, dans lequel au moins environ 10% (poids/poids) de la clindamycine cristalline, à l'état de base libre, isolée, est de Forme III.

37. Procédé selon la revendication 36, dans lequel la clindamycine cristalline, à l'état de base libre, isolée, est **caractérisée par** un motif de diffraction des rayons X sur poudre sensiblement tel que représenté à la Figure 9.

38. Procédé selon la revendication 36, dans lequel la clindamycine cristalline, à l'état de base libre, isolée, est **caractérisée par** une calorimétrie différentielle à balayage manifestant un endotherme ayant une température de début extrapolée d'environ 64,4°C, une température de pic d'environ 69,4°C et une chaleur associée d'environ 69,4 J/g.
